Europäisches Patentamt
European Patent Office
Office européen des brevets

(11) Publication number: **0 405 983 A2**

# EUROPEAN PATENT APPLICATION

(21) Application number: 90307100.9

(22) Date of filing: 28.06.90

(51) Int. Cl.5: C07D 309/30, C09K 19/34

(30) Priority: 28.06.89 JP 166295/89

(43) Date of publication of application: 02.01.91 Bulletin 91/01

(84) Designated Contracting States: DE FR GB

(71) Applicant: MITSUBISHI RAYON CO., LTD
3-19, Kyobashi 2-chome Chuo-ku
Tokyo(JP)

(72) Inventor: Hayashi, Seiji, C/o Tokyo Research Laboratories
Mitsubishi Rayon Co. Ltd., Noborito 3816
Tama-ku
Kawasaki-shi, Kanagawa 241(JP)
Inventor: Sakashita, Keiichi, C/o Tokyo Research Laboratorie
Mitsubishi Rayon Co. Ltd., Noborito 3816
Tama-ku
Kawasaki-shi, Kanagawa 241(JP)
Inventor: Ikemoto, Tetsuya, C/o Tokyo Research Laboratorie
Mitsubishi Rayon Co. Ltd., Noborito 3816
Tama-ku
Kawasaki-shi, Kanagawa 241(JP)
Inventor: Sako, Yoshihiro, C/o Tokyo Research Laboratorie
Mitsubishi Rayon Co. Ltd., Noborito 3816
Tama-ku
Kawasaki-shi, Kanagawa 241(JP)

(74) Representative: Lawrence, Peter Robin Broughton et al
GILL JENNINGS & EVERY 53-64 Chancery Laneane
London WC2A 1HN(GB)

(54) **Optically active substance and liquid crystal composition.**

(57) An optically active substance having a δ-valerolactone ring is described which is represented by the formula (1):

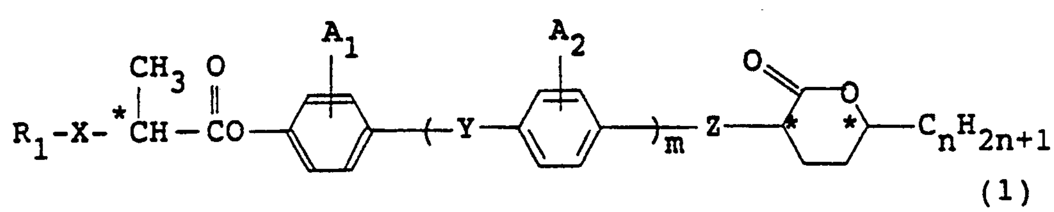

(1)

wherein X is -O-, $-OCH_2-$ or $-SCH_2-$, $R_1$ is C1 - 18 alkyl, n is an integer of from 1 to 14, m is 1 or 0, Y is a direct bond, -OCO-, $-CO_2-$, $-CH_2O$ or $-OCH_2-$, Z is -O-, $-CO_2-$ or $-CH_2O$, $A_1$ and $A_2$ are H, F, Cℓ or cyano, and the carbon atom marked with "*" indicates the asymmetric carbon atom. This optically active substance is used alone or as a mixture thereof with other optically active substances.

EP 0 405 983 A2

Fig.1

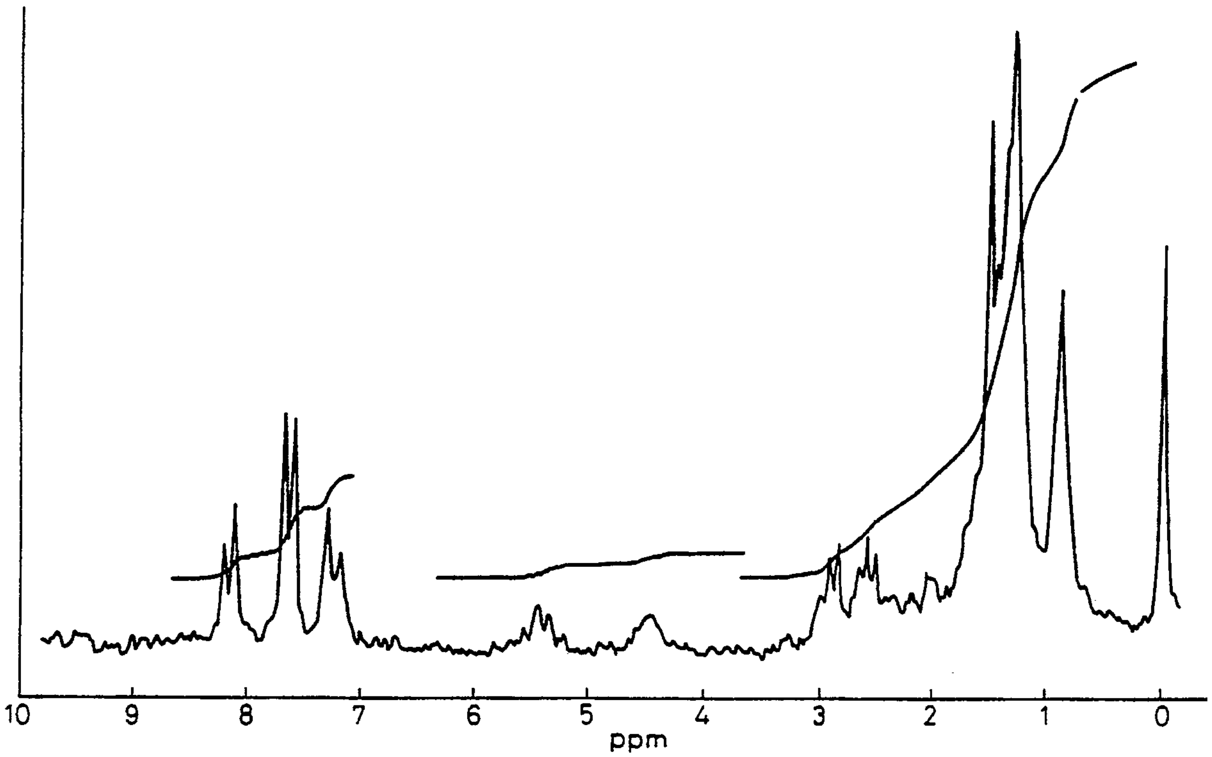

# OPTICALLY ACTIVE SUBSTANCE AND LIQUID CRYSTAL COMPOSITION

The present invention relates to a novel optically active substance and a liquid crystal composition comprising this optically active substance.

Liquid crystals currently used in a liquid crystal display are classified into the nematic phase, and since they are of the light-receiving type, they are characterized in that they do not cause eye fatigue and have a very small power consumption. Nevertheless, these liquid crystals have problems in that the response speed is low and the view angle of the display is narrow.

Display devices and printer heads using a ferroelectric liquid crystal having advantageous characteristics similar to those of the nematic liquid crystal, such as the property of not causing eye fatigue and a small power consumption, and having a high response speed and high contrast characteristics comparable to those of a light-emitting type display element, have been investigated.

The discovery of the ferroelectric liquid crystal was discovered for the first time by R.B. Meyer et al [J. Physique, 36 , L-69 (1975)]. This ferroelectric liquid crystal is classified into the one exhibiting a chiral smectic C phase (hereinafter referred to as "Sm*C phase"), and a typical instance of the ferroelectric liquid crystal is p-decyloxybenzylidene-p′-amino-2-methylbutyl cinnamate (hereinafter referred to as "DOBAMBC") represented by the following formula (2):

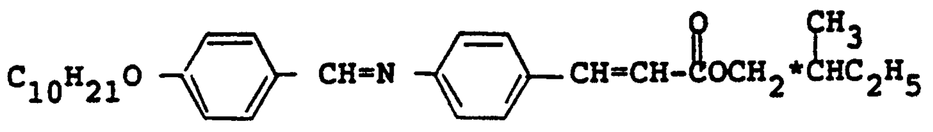

(2)

In DOBAMBC and most of the ferroelectric liquid crystals proposed thereafter, the range of temperatures showing the ferroelectric property (the range of temperatures wherein the Sm*C phase is present) is very narrow, and these liquid crystal materials cannot be practically used alone. Therefore, attempts have been made to expand the range of temperatures showing the Sm*C phase to the lower and higher temperature sides, taking room temperature as the center, by mixing a variety of ferroelectric liquid crystals. A ferroelectric liquid crystal having a larger spontaneous polarization than heretofore proposed ferroelectric liquid crystals is desired for a printer head for which a very short response time is required.

Nevertheless, in the case of mixtures of ferroelectric liquid crystals having an Sm*C phase, the kinds of compounds (liquid crystals) to be mixed are limited, and a liquid crystal mixture having satisfactory performances is currently difficult to obtain. Moreover, a compound having a Schiff base, such as DOBAMBC, has a poor light stability and is readily colored.

Accordingly, the inventors carried out investigations with a view to finding a component to be incorporated into a liquid crystal composition, which is capable of increasing the spontaneous polarization of the liquid crystal composition without substantially increasing the viscosity of the liquid crystal composition, and as a result, the inventors have completed the present invention.

A primary object of the present invention is to provide an optically active substance which is chemically stable and has an excellent light stability, and which gives a large spontaneous polarization to a liquid crystal composition when incorporated therein, and a liquid crystal composition comprising this optically active substance.

More specifically, in accordance with the present invention, there is provided an optically active substance having a δ-valerolactone ring, which is represented by the following general formula (1):

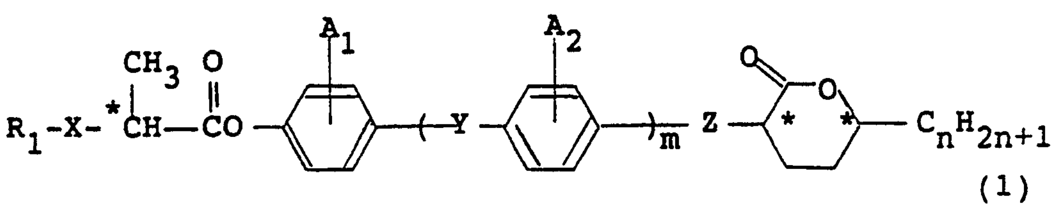

(1)

wherein X represents -O-, $-OCH_2-$ or $-SCH_2-$, $R_1$ represents an alkyl group having 1 to 18 carbon atoms, n is an integer of from 1 to 14, m is 1 or 0, Y represents a direct bond, -OCO-, $-CO_2-$, $-CH_2O-$ or $-OCH_2-$, Z represents -O-, $-CO_2-$ or $-CH_2O$, $A_1$ and $A_2$ independently represent hydrogen, fluorine, chlorine or a cyano

group, and the carbon atom marked with "*" indicates an asymmetric atom. -OCO- and $-CO_2-$ refer to $-O-\overset{O}{\overset{\|}{C}}-$ and $-\overset{O}{\overset{\|}{C}}-O-$ respectively.

In accordance with the present invention, there is further provided a liquid crystal composition comprising an optically active substance having a δ-valerolactone ring, which is represented by the general formula (1).

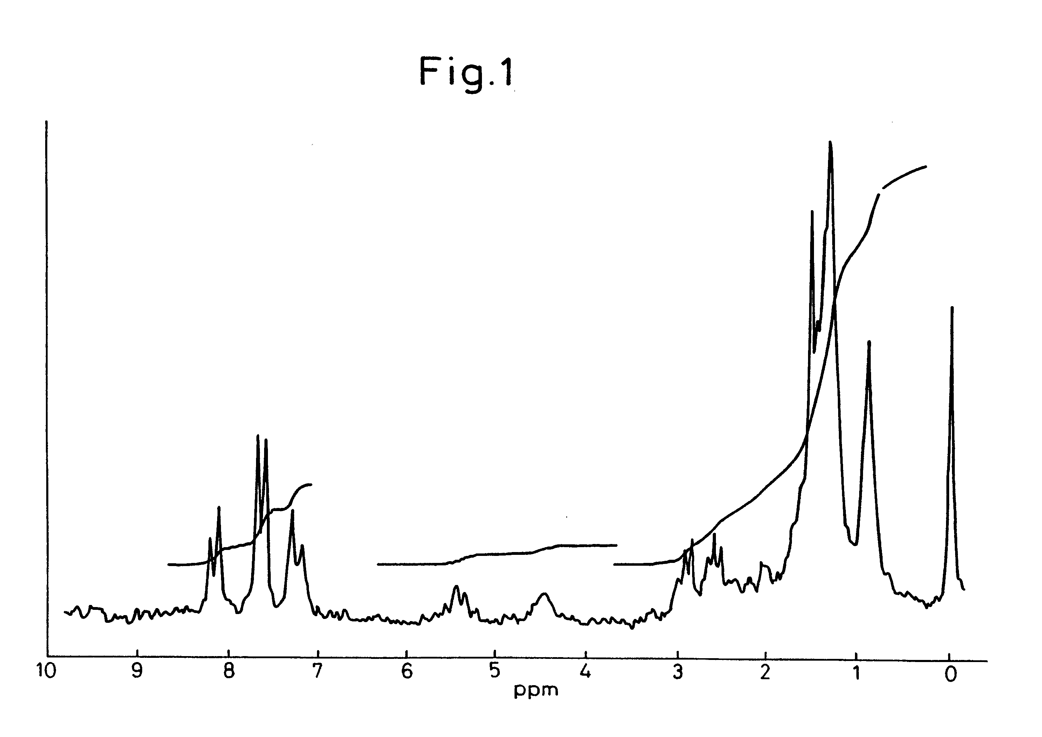

Fig. 1 shows the NMR spectrum of the optically active substance obtained in Example 1 of the present invention.

In the optically active substance represented by formula (1), if n is 15 or larger, purification of the optically active lactone is relatively difficult, and if the carbon number of $R_1$ is 19 or larger, purification of the alkyl bromide used is relatively difficult and the productivity is reduced. Furthermore, when the substance having such $R_1$ is mixed with other liquid crystals, a tendency toward a reduction of the spontaneous polarization is observed.

The synthesis of the optically active substance of the present invention will now be described.

A) Preparation of Optically Active 3-Alkylthio-2-methylpropionic Acid

An optically active 3-alkylthio-2-methylpropionic acid is synthesized by using D-(-)-β-acetylthio-α-methylpropionic acid or methyl L-(+)-β-acetylthio-α-methylpropionate derived from methyl methacrylate as the starting material. The synthesis process using methyl L-(+)-β-acetylthio-α-methylpropionate is represented by the following reaction formula:

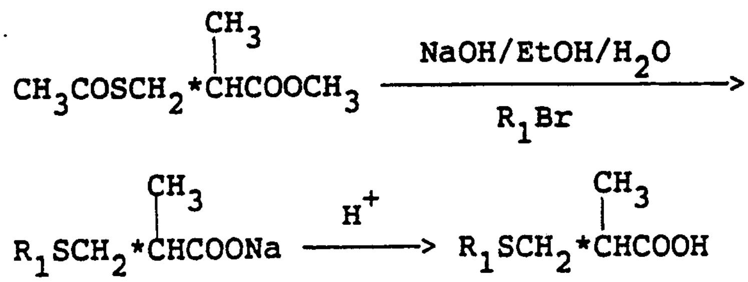


Also, an optically active 3-alkylthio-2-methylpropionic acid can be similarly prepared by using D-(-)-β-acetylthio-α-methylpropionic acid.

B) Preparation of Optically Active 3-Alkoxy-2-methylpropionic Acid

In the following description DHP represents dihydropyran, THP represents tetrahydropyran, LAH represents lithium-aluminum hydride and THF represents tetrahydrofuran.

An optically active 3-alkoxy-2-methylpropionic acid can be synthesized by using optically active ethyl β-hydroxyisobutyrate as the starting material, according to the following synthesis route:

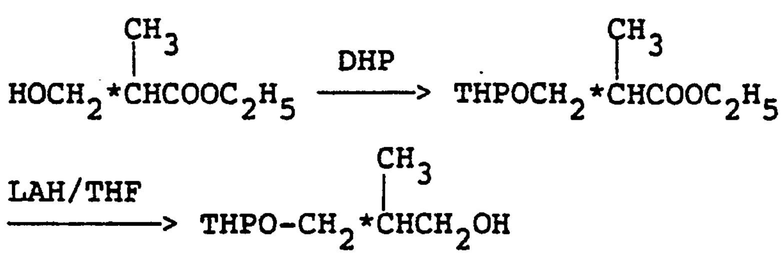

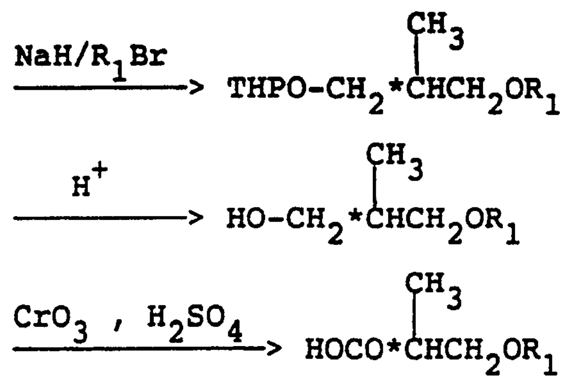


C) Preparation of Optically Active 2-Alkoxypropionic Acid

An optically active 2-alkoxypropionic acid can be easily prepared by reacting optically active ethyl lactate with silver oxide and an alkyl bromide in N,N′-dimethylformamide, to obtain an optically active ethyl 2-alkoxypropionate, and hydrolyzing the obtained compound.

Furthermore a 3-hydroxy-6-alkyl-δ-valerolactone can be obtained according to the following process.

Namely, first an optically active β-hydroxycarboxylic acid is prepared, for example, by reacting a methyl alkyl ketone with diethyl carbonate in the presence of sodium hydride to form an ethyl β-ketocarboxylate, hydrolyzing this ester with potassium hydroxide and subjecting the carbonyl group at the β-position to an asymmetric reduction with baker's yeast, as represented by the following reaction formula:

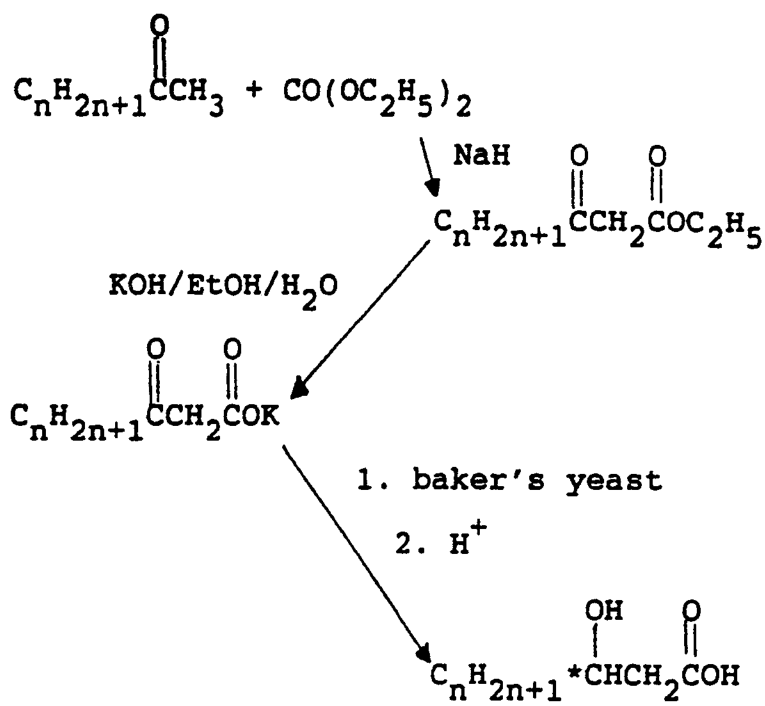


When the carbon number (n) in the alkyl group of the methyl alkyl ketone is 3 or smaller, preferably an alkyl ester of a β-ketocarboxylic acid having a larger carbon number (i.e., at least 6) is reduced instead of the ethyl β-ketocarboxylate, because the optical yield can be thereby further increased.

Separately, S-(-)-malic acid is reacted with acetyl chloride, and the obtained product is reacted with anhydrous ethanol to obtain optically active monoethyl α-acetoxymalate, as represented by the following reaction formula [see Tetrahedron, 41 , No. 13, 2751-2758 (1985)]:

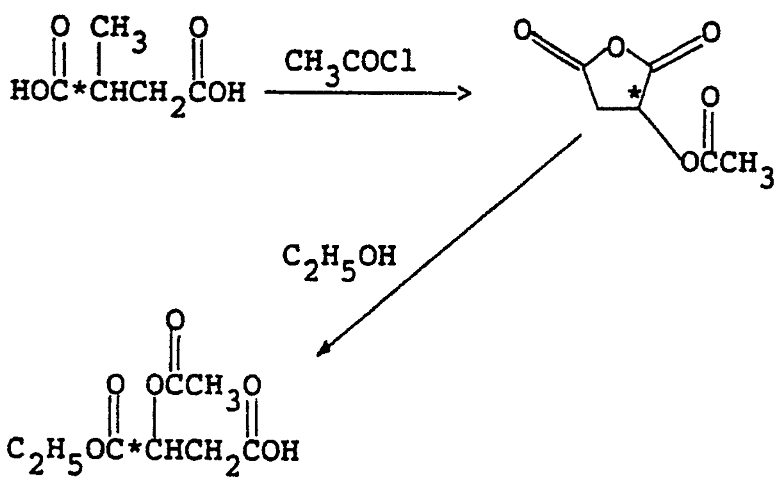


The obtained optically active β-hydroxycarboxylic acid and the monoethyl ester of the optically active α-acetoxy-dibasic acid are subjected to the Kolbe electrolysis process, as represented by the following reaction formula:

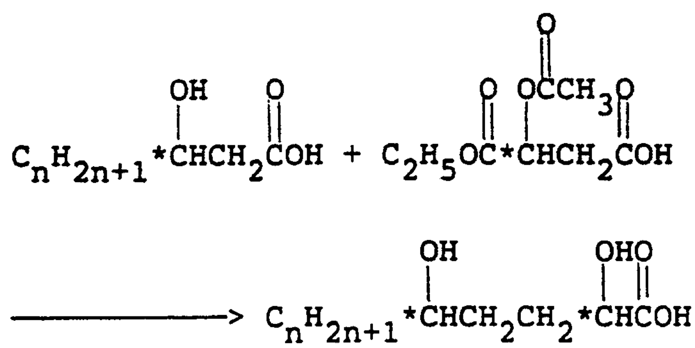

The above-mentioned valerolactone derivative can be obtained by cyclizing the obtained product in the presence of p-toluenesulfonic acid, as represented by the following reaction formula:

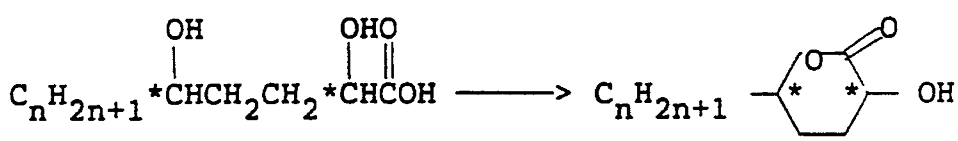

[Synthesis of Compound Represented by General Formula (1)]

The compound represented by the general formula (1) can be synthesized according to the following route.

The description will be made with reference to an example where R* is

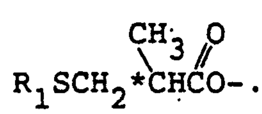

Compounds where R* is another optically active group can be synthesized according to a similar route.

In the following description, DCC represents dicyclohexylcarbodiimide and $PPh_3$ represents triphenylphosphine.

(i) In the case of m = 0

(a) Where Z = $-\overset{O}{\overset{\|}{C}}O-$

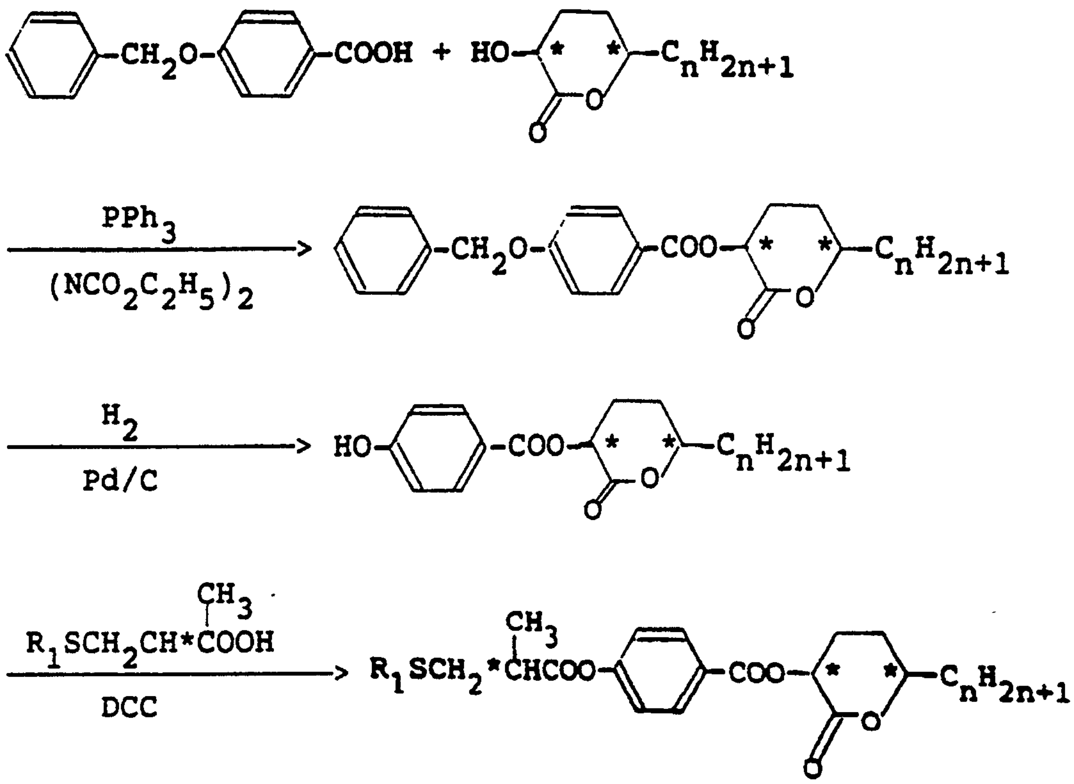


(b) Where Z = -O-

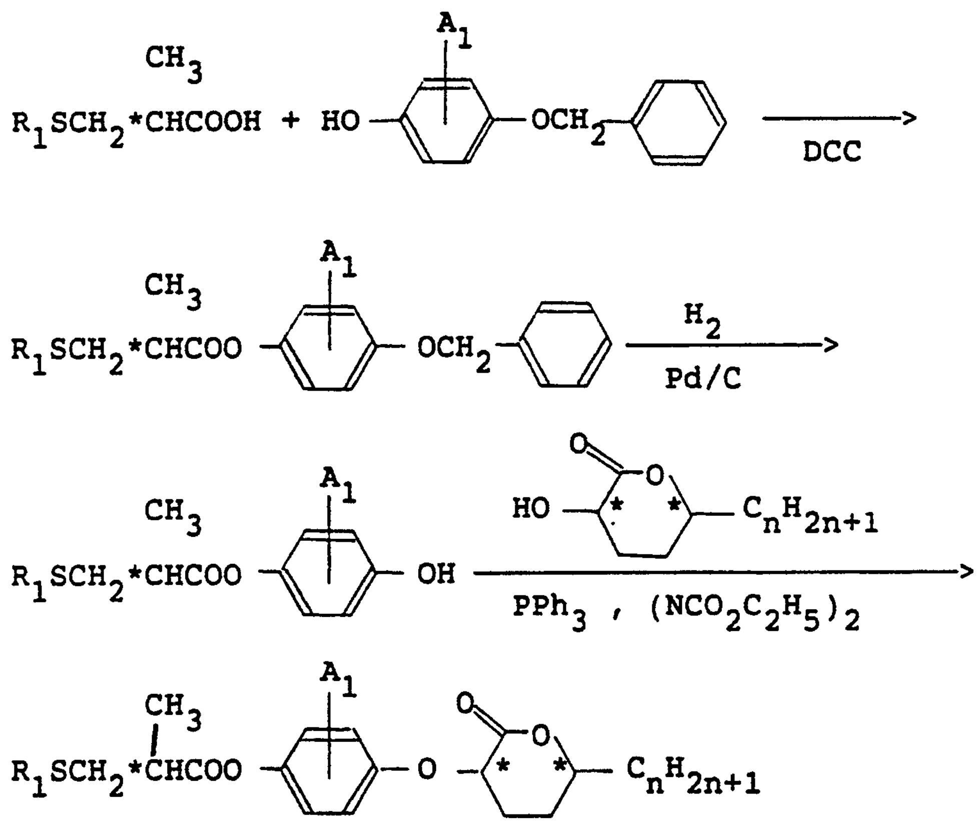

(c) Where Z = $-CH_2O-$

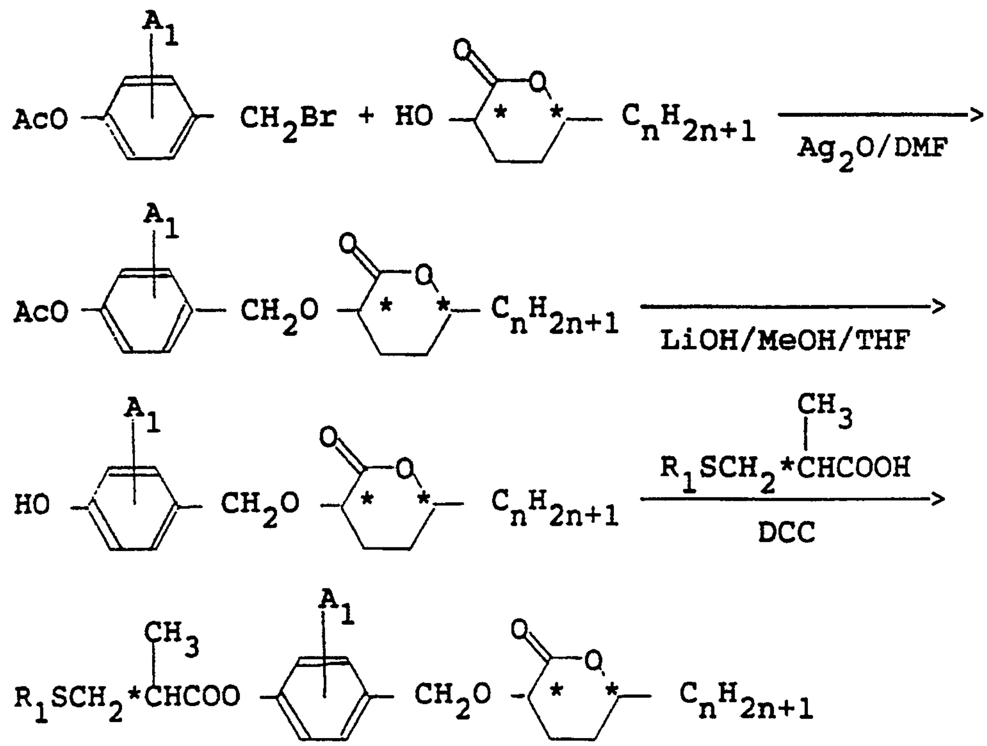


(ii) In the case of m = 1 and Y is not direct bond

(a) Where Y = -COO-

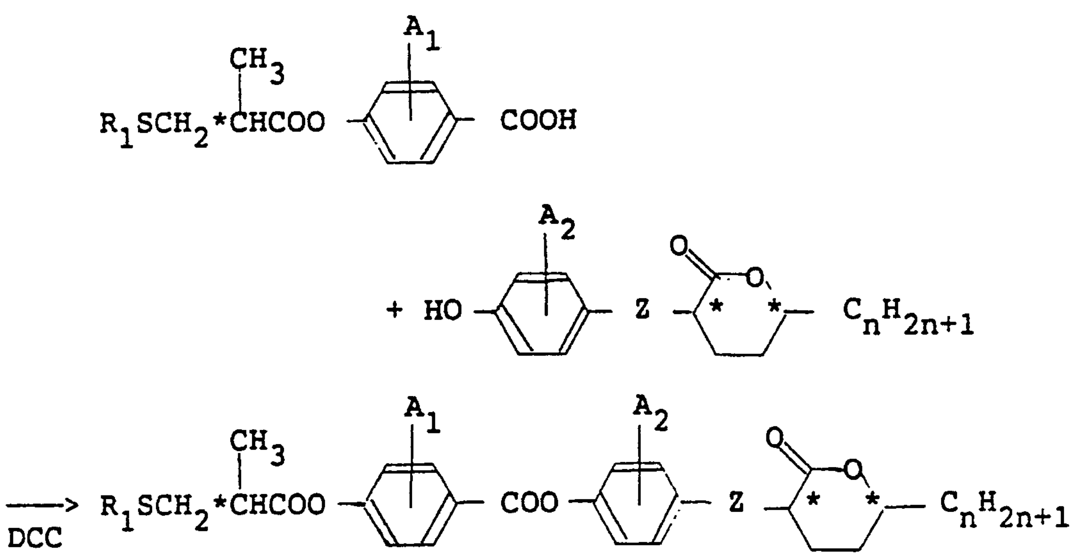


(b) Where Y = -OCO-

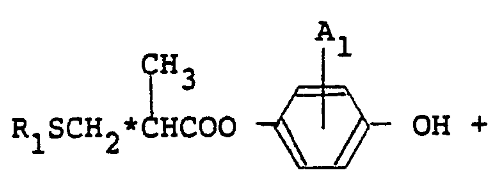

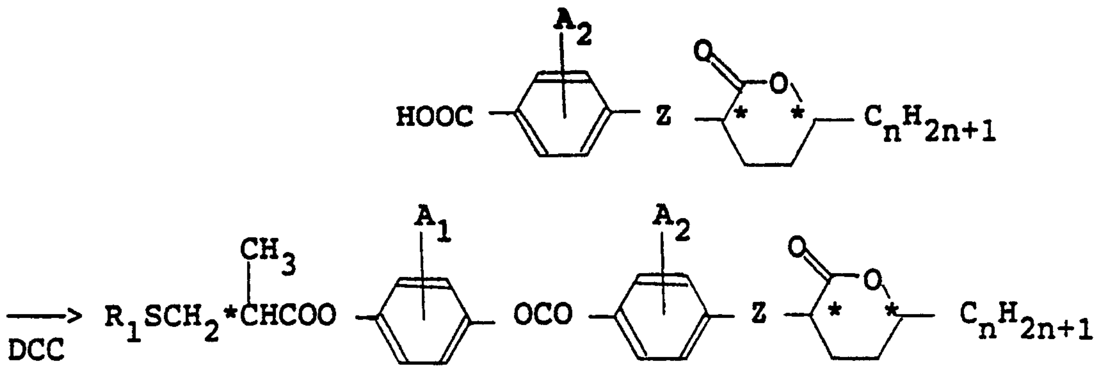


(c) Where Y = $-CH_2O-$

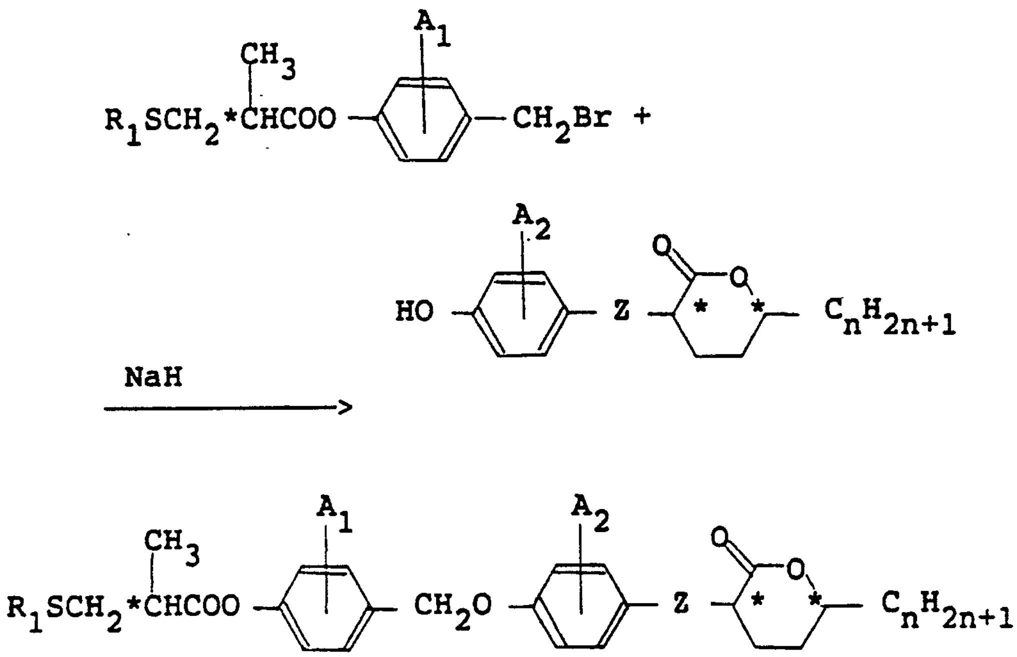


(d) Where Y = $-OCH_2-$

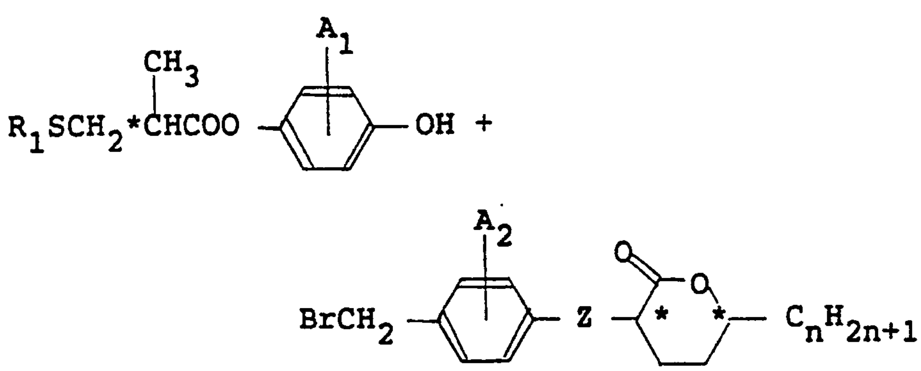

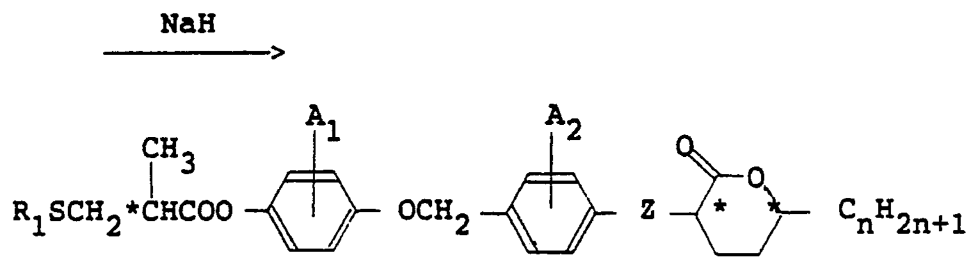


(iii) In the case of m = 1 and Y = direct bond

(a) Where Z = -COO-

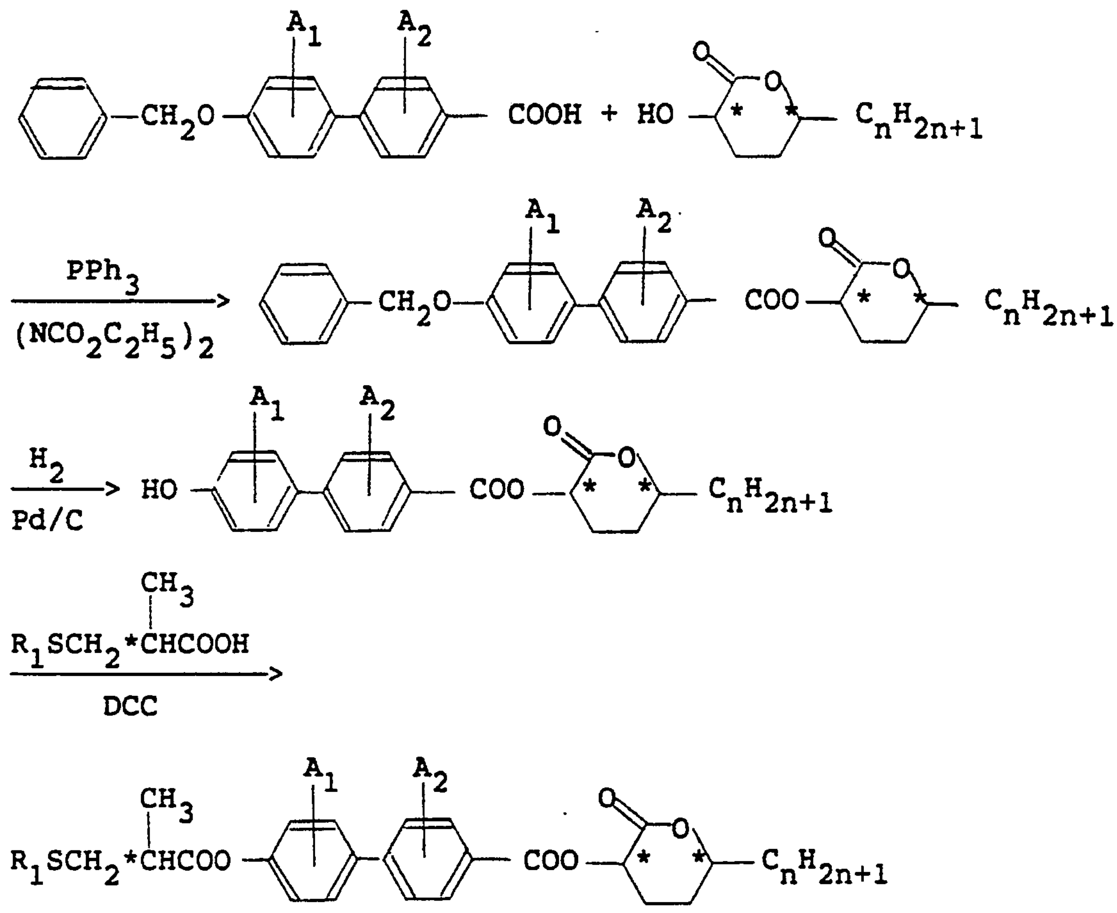


(b) Where Z = -O-

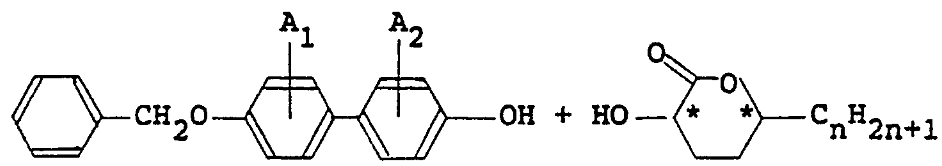

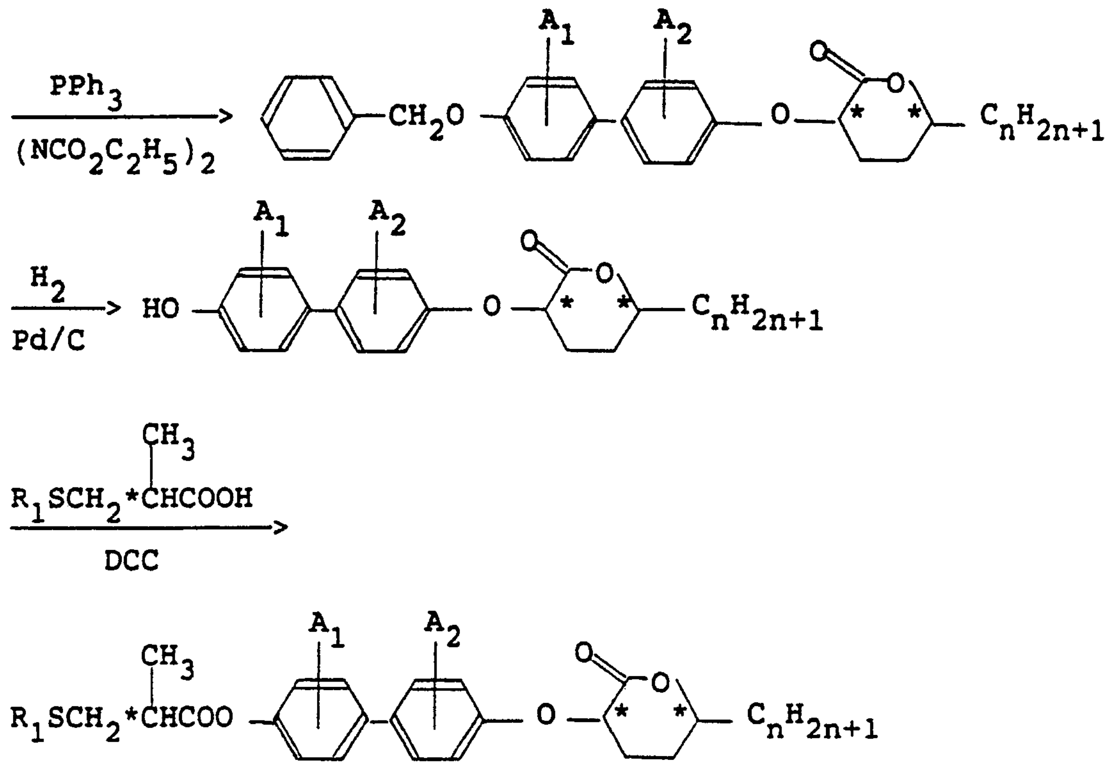


(c) Where Z = $-CH_2O-$

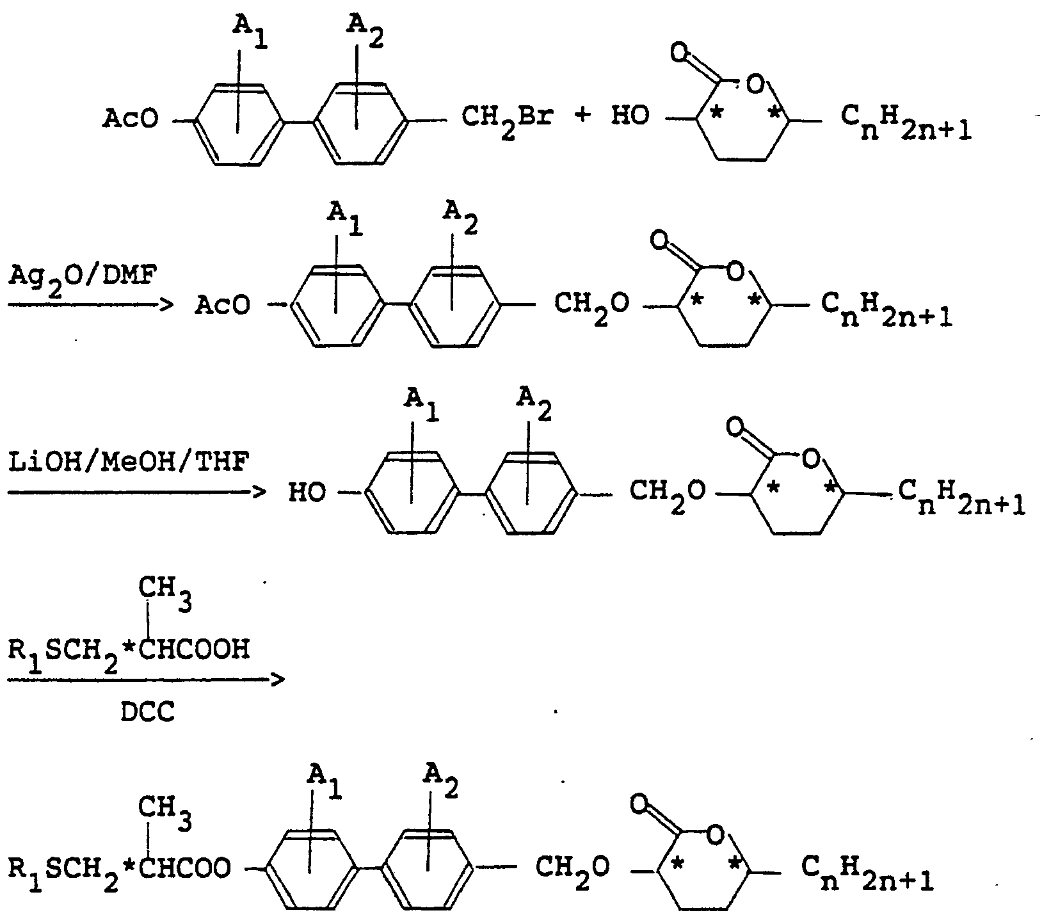

Since the compound of the present invention does not contain an azomethine bond possessed by the conventional ferroelectric liquid crystal compounds, the chemical stability such as the hydrolysis resistance is improved and the light stability is better than that of the cinnamic acid type compounds. Accordingly, the compound of the present invention has good characteristics as a display material.

Some of the compounds included within the scope of the present invention do not show the chiral smectic C phase (Sm*C phase) when used alone, but when these compounds are added to other liquid crystal materials having the SmC phase, they show an effect of inducing the Sm*C phase.

In the compound of the present invention, the following effects can be attained by the synergistic action of the δ-valerolactone ring and the optically active branched alkyl chain.

(1) By adjusting the spontaneous polarization originating in the δ-valerolactone ring and the spontaneous polarization originating in the optically active branched alkyl chain, the intensity of the spontaneous polarization can be varied without substantially changing the phase series or thermal properties.

(2) By increasing the spontaneous polarization, the response time can be shortened.

(3) By adjusting the absolute arrangement of the optically active groups, the helical pitch of the Ch phase and Sm*C phase can be adjusted.

The liquid crystal composition of the present invention will now be described.

The liquid crystal composition of the present invention comprises at least one compound represented by formula (1). A composition comprising a plurality of ferroelectric liquid crystals compounds, optionally with other additive compounds, is advantageous over a composition comprising one ferroelectric liquid crystal compound alone, because the range of applicable temperatures can be broadened.

As examples of other ferroelectric liquid crystal compounds that can be mixed with at least one compound represented by formula (1), compounds having a structure shown below can be used:

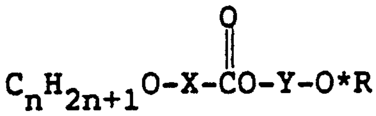 [n = 7 - 12, X = 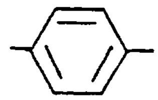 ,

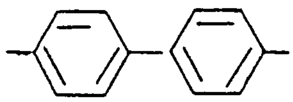 , 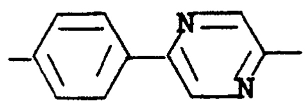 , 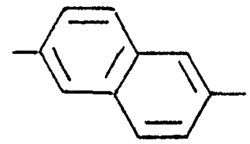 or

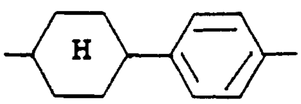 , Y = 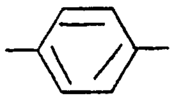 , 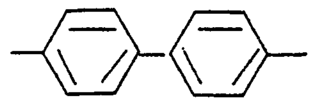 ,

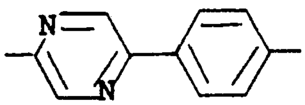 , 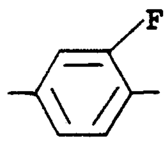 or 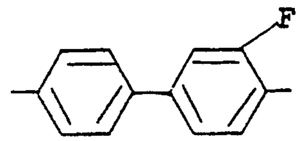 ,

*R = 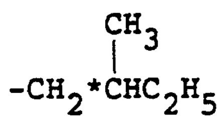 , 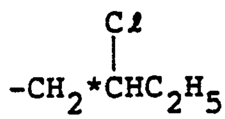 , 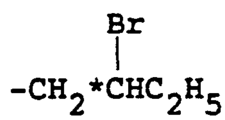 ,

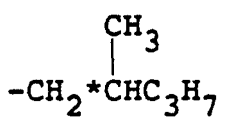 , 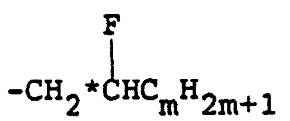 (m = 5, 6, 8, 10 or 12),

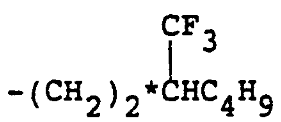 , 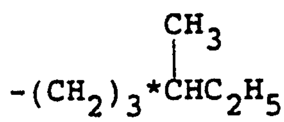 , 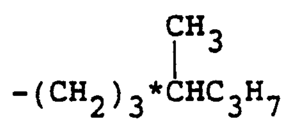 ,

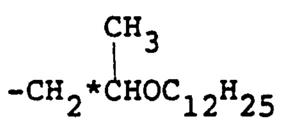 , 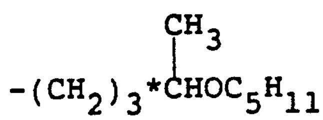 , 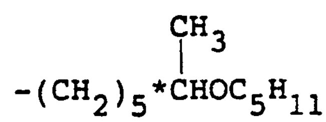 ,

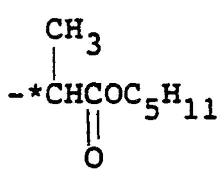 or 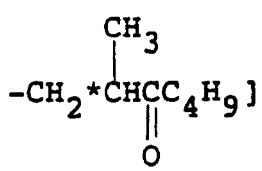 ],

$C_nH_{2n+1}O\text{-X-O}\overset{O}{\overset{\|}{C}}\text{-Y-O*R}$ [n = 6 - 10,

X = 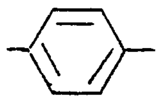 , 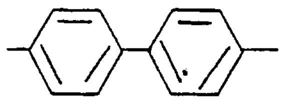 or 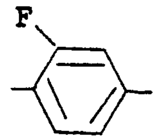 ,

Y = 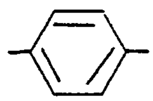 or 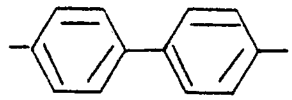 ,

*R = $-CH_2{*}\overset{CH_3}{\overset{|}{C}}HC_2H_5$ , $-{*}\overset{CH_3}{\overset{|}{C}}HC_6H_{13}$ , $-(CH_2)_3{*}\overset{CH_3}{\overset{|}{C}}HC_2H_5$ ,

$-CH_2{*}\overset{CH_3}{\overset{|}{C}}HCH(CH_3)_2$ , $-CH_2{*}\overset{F}{\overset{|}{C}}HC_8H_{17}$ , $-CH_2{*}\overset{CH_3}{\overset{|}{C}}HOC_5H_{11}$ ,

$-{*}\overset{CH_3}{\overset{|}{C}}H\underset{O}{\underset{\|}{C}}OC_8H_{17}$ or $-\underset{O}{\underset{\|}{C}}{*}\overset{CH_3}{\overset{|}{C}}HOC_8H_{17}$],

$C_nH_{2n+1}\text{-X-}\overset{O}{\overset{\|}{C}}\text{-O-Y-O*R}$ [n = 3, 7 or 8,

X = 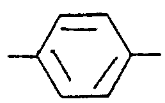 , 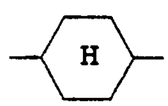 , 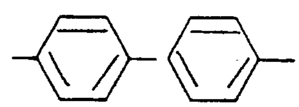 ,

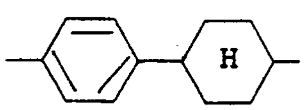 , 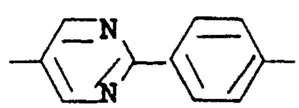 or

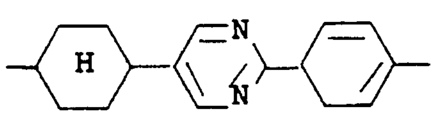 , Y = 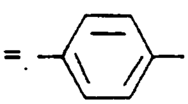 ,

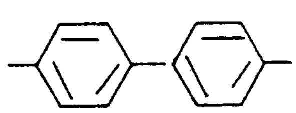 or 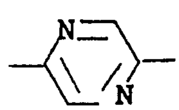 ,

*R = $-CH_2{*}\overset{CH_3}{\overset{|}{C}}HC_mH_{2m+1}$ (m = 2 or 6) or

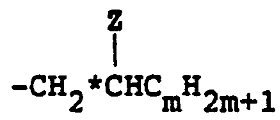 (m = 5, 6 or 8, Z = F or Cℓ)],

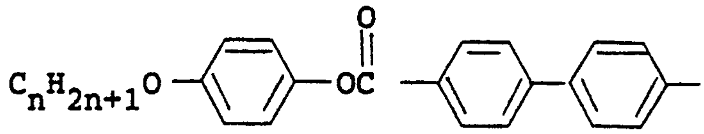 [n = 8, 10, 12 or 16, *R = 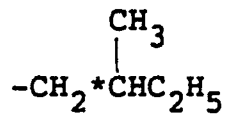 , 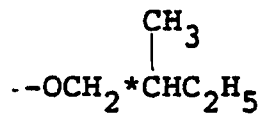 ,

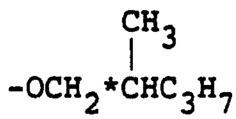 , 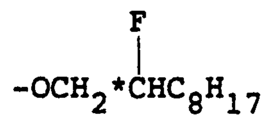 , 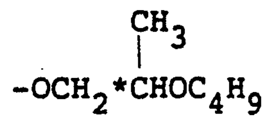

or 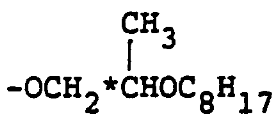 ],

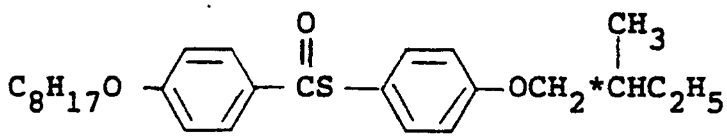 ,

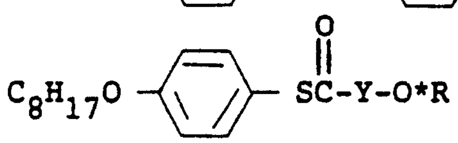 [Y = 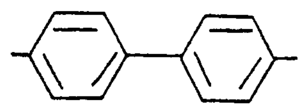 or

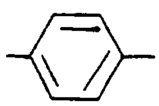 , *R = 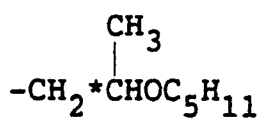 or 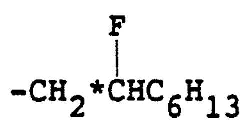 ],

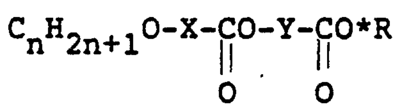 [n = 5 - 8, 10 or 12, X and Y =

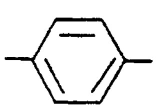 , 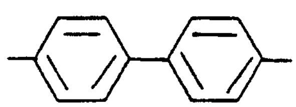 or 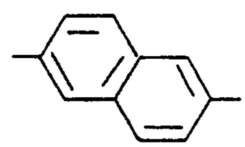 ,

with the proviso that at least one of X and Y is 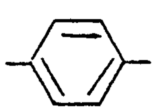 , *R = 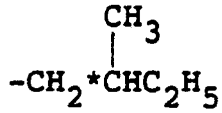 , 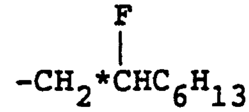 ,

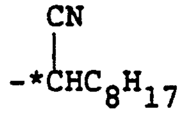 , 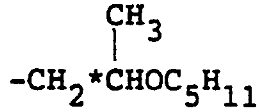 , 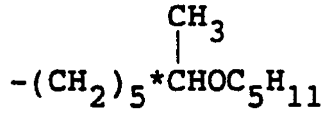 ,

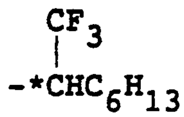 , 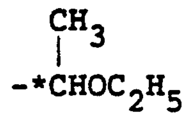 or 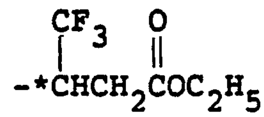 ],

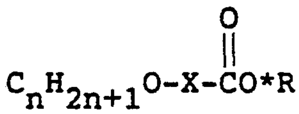 [n = 5, 7, 8 or 12, X = 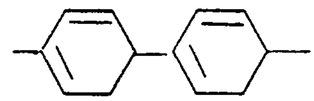 ,

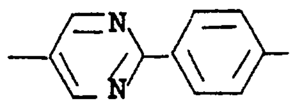 , 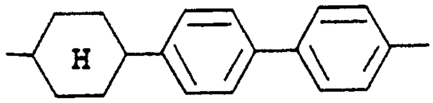 ,

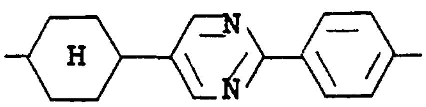 or 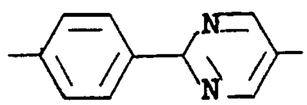 ,

*R = 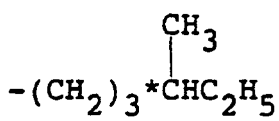 , 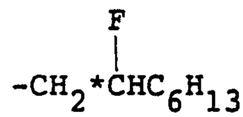 ,

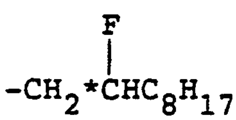 or 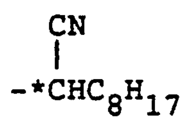 ],

R′-X-O*R [R′ = $-C_8H_{17}$ , $-C_9H_{19}$ , $-C_{10}H_{21}$ , $-OC_{10}H_{21}$ or $-C_{11}H_{23}$ , X = 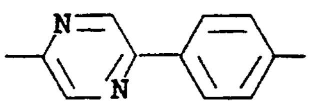 or 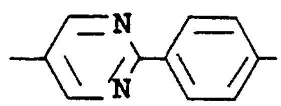 ,

*R = 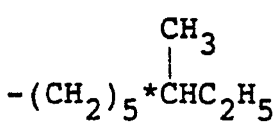 , 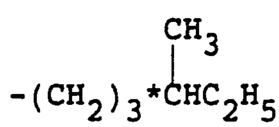 , 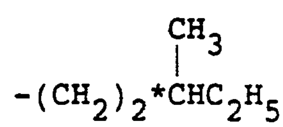 ,

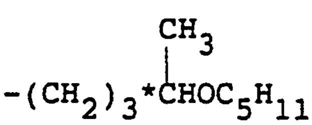 , 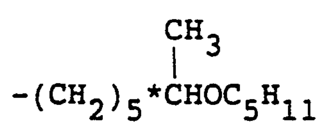 ,

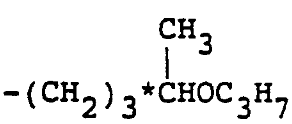 , 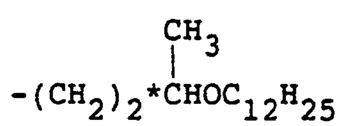 ,

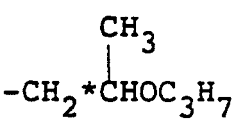 , 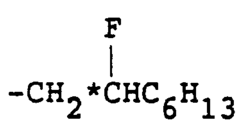 , 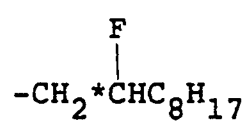

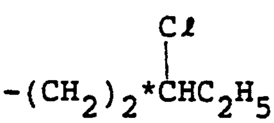 , 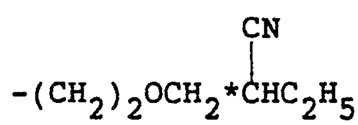 ,

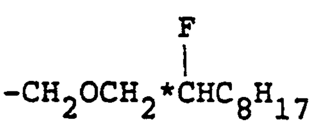 , or 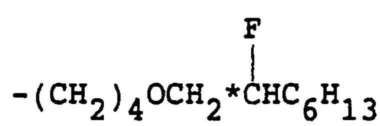 ],

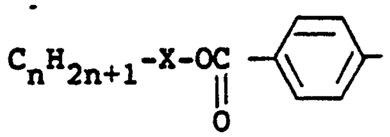 [n = 8, 10 or 12,

X = 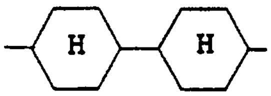 or 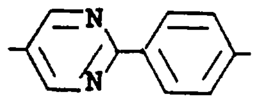,

*R = $-(CH_2)_3{*}\overset{CH_3}{\overset{|}{C}}HC_2H_5$ , $-(CH_2)_2{*}\overset{CH_3}{\overset{|}{C}}HC_2H_5$ , $-(CH_2)_2{*}\overset{Cl}{\overset{|}{C}}HC_2H_5$ ,

$-CH_2{*}\overset{F}{\overset{|}{C}}HC_6H_{13}$ or $-CH_2{*}\overset{F}{\overset{|}{C}}HC_4H_7$],

R'-X-$OCH_2$Y-*R [R' = $-C_2H_5$ , $-C_{10}H_{21}$ , $-OC_6H_{13}$ , $-OC_7H_{15}$ , $-OC_8H_{17}$ , $-OC_{10}H_{21}$ or $-OC_{12}H_{25}$ , X = 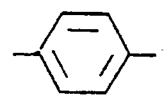 ,

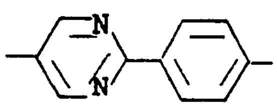 , 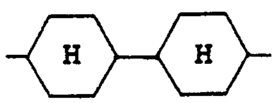 or 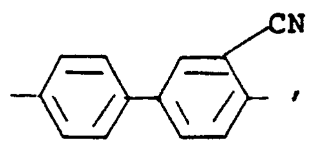 ,

Y = 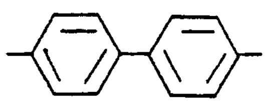 , 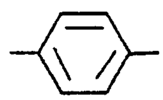 or

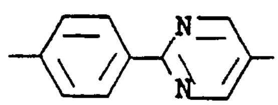 , *R = $-CH_2{*}\overset{CH_3}{\overset{|}{C}}HC_2H_5$ , $-O{*}\overset{CH_3}{\overset{|}{C}}HOC_3H_7$ ,

$-OCH_2{*}\overset{CH_3}{\overset{|}{C}}HC_2H_5$ , $-O(CH_2)_5{*}\overset{CH_3}{\overset{|}{C}}HOC_3H_7$ , $-\overset{O}{\overset{||}{C}}O(CH_2)_2{*}\overset{CH_3}{\overset{|}{C}}HC_2H_5$ ,

$-O{*}\overset{CH_3}{\overset{|}{C}}H\underset{\underset{O}{||}}{C}OC_6H_{13}$ , $-O(CH_2)_2{*}\overset{Cl}{\overset{|}{C}}HC_2H_5$ , $-OCH_2{*}\overset{F}{\overset{|}{C}}HC_4H_9$ ,

$-CH_2CH_2\overset{O}{\overset{||}{C}}OCH_2{*}\overset{F}{\overset{|}{C}}HC_4H_9$ or $-OCH_2{*}\overset{F}{\overset{|}{C}}HC_6H_{13}$],

R'-X'$CH_2$O-Y-*R [R' = $C_{10}H_{21}O-$ or $C_nH_{2n+1}-$ (n = 4, 6, 7, or 8), X = 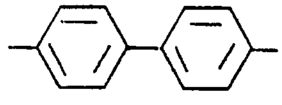 , 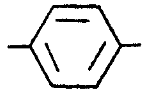 ,

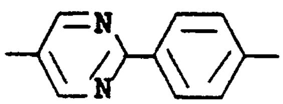 , 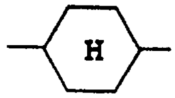 or 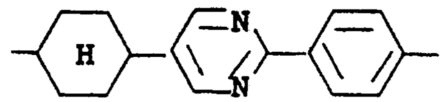 ,

Y = 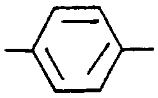 , 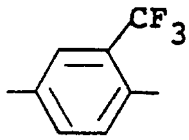 or 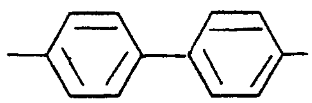 ,

*R = 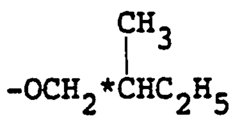 , 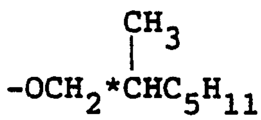 , 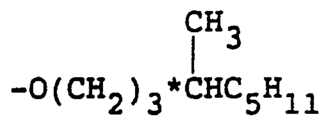 ,

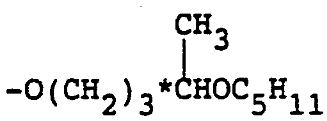 , 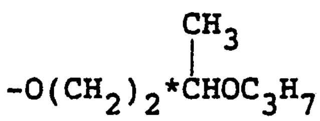 , 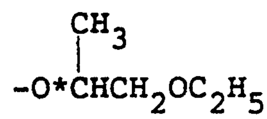 ,

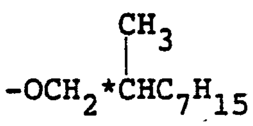 , 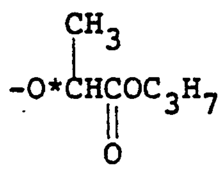 , 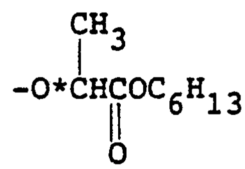 ,

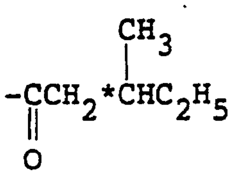 , 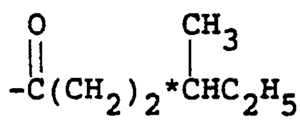 , 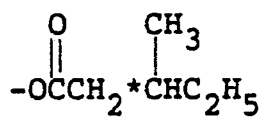 ,

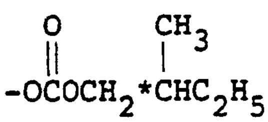 , 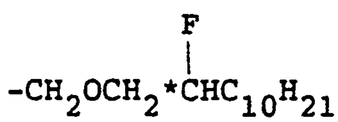 , 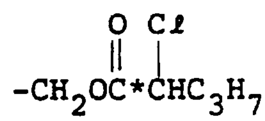 ,

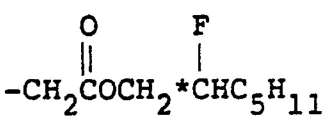 or 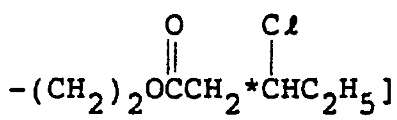],

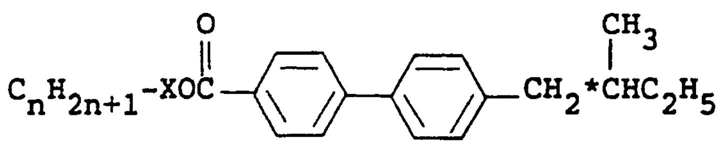

[n = 6 or 8, X = 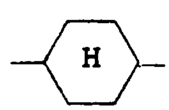 , 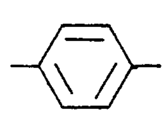 or 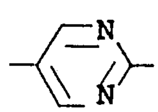 ],

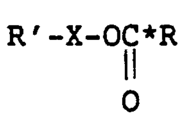 [R' = $-C_{10}H_{21}$ , $-OC_{10}H_{21}$ or $-OC_8H_{17}$ ,

X = 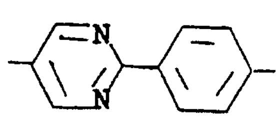 or 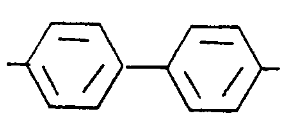 , *R = 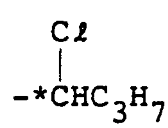 ,

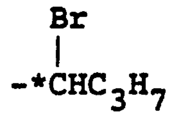 , 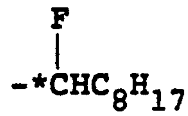 , 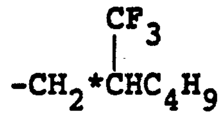 , 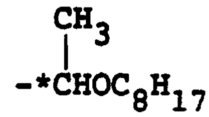

or 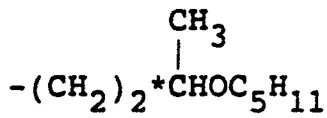 ],

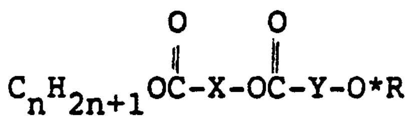 [n = 6 or 10,

X = 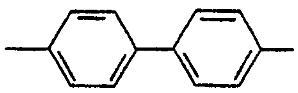 , 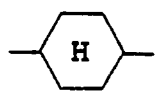 or 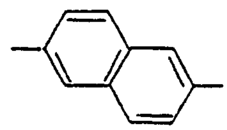 ,

Y = 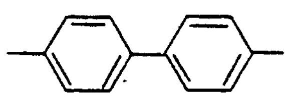 , 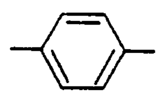 or 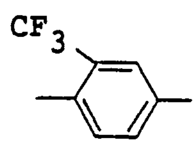 ,

*R = 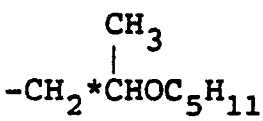 , 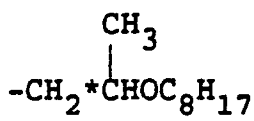 , 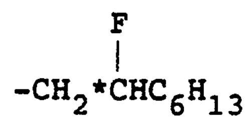 or

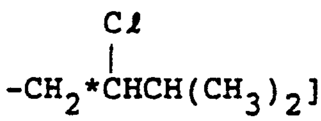 ],

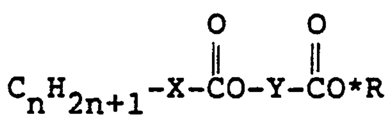 [n = 5 or 8,

X = 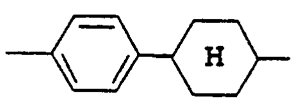 or 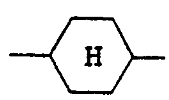 , Y = 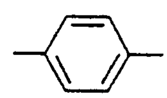 or

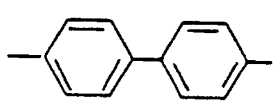 , *R = 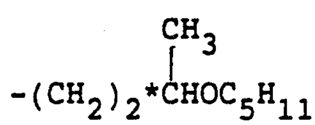 or 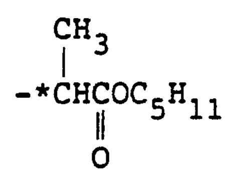 ],

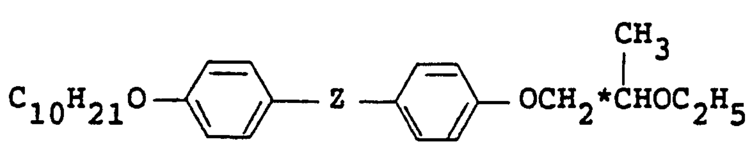 [Z = $-(CH_2)_2-$,

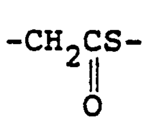 or 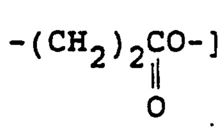 ],

$C_nH_{2n+1}$-X-CO-Y-*R [n = 8, 10, or 12, X = 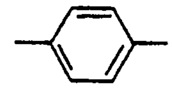 ,

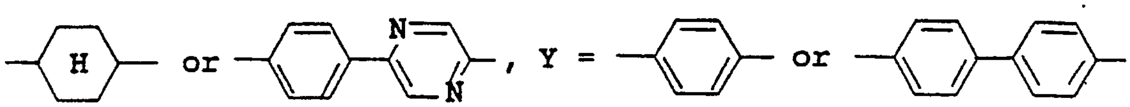

*R = $-CH_2{*}CH(CH_3)C_2H_5$ , $-CH_2{*}CHFC_6H_{13}$ or $-OCO{*}CHClC_2H_5$],

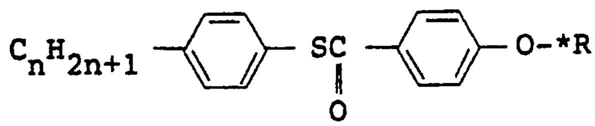

[n = 8 or 10,

*R = $-CH_2{*}CHFC_8H_{17}$ or $-CH_2{*}CH(CH_3)C_3H_7$],

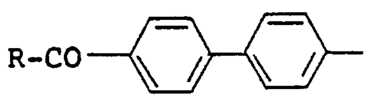

[R = $-C_8H_{17}$ or $-CH(CH_3)C_3H_7$ ,

Z = -O- or -CO(=O), *R' = $-CH_2{*}CHFC_8H_{17}$ or

$-CH_2{*}CHClCH(CH_3)_2$],

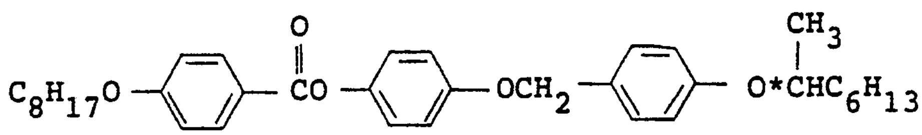

and

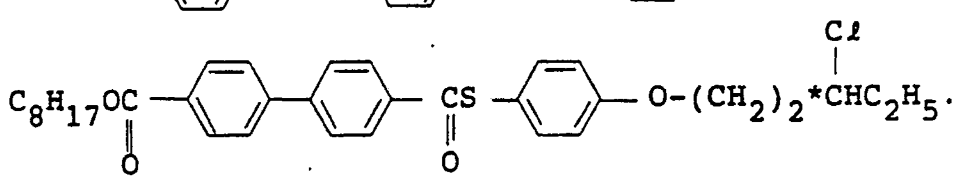

Other ferroelectric liquid crystal compounds can be used in the state mixed with the compound of formula (1).

Furthermore, when the compound of the present invention is mixed with a non-chiral liquid crystal compound showing the SmC phase or a composition comprising a non-chiral liquid crystal compound, the mixture is a ferroelectric liquid crystal. Furthermore, a mixture having a short response time can be obtained by mixing a small amount of the compound of the present invention with a non-chiral liquid crystal composition, and thus the compound of the present invention is very valuable. The non-chiral crystal compound that can be mixed with the compound of the present invention, there can be mentioned compounds represented by the following formula:

$C_mH_{2m+1}$-X-$C_nH_{2n+1}$ (m and n = 4 - 16,

X = 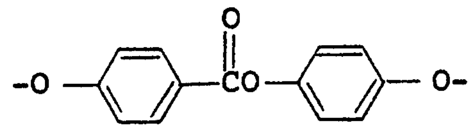 , 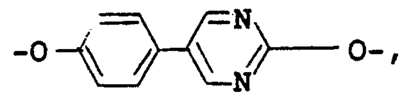 ,

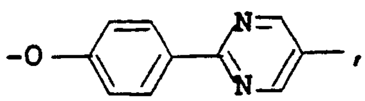 , 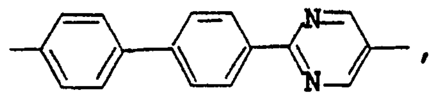 ,

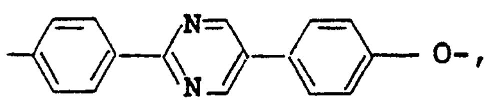 , 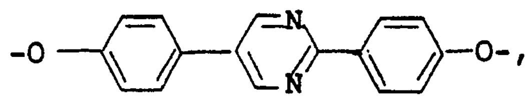 ,

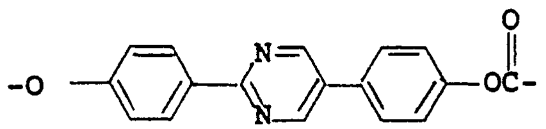

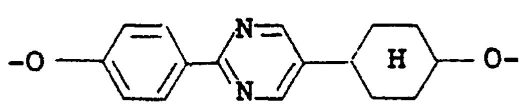 or 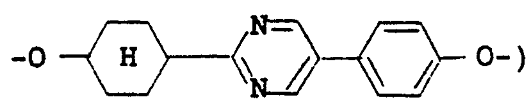 ).

Other non-chiral liquid crystal compounds showing the SmC phase can be used in the state mixed with the compound of formula (1).

The liquid crystal composition of the present invention is characterized in that the zigzag defects often observed in conventional ferroelectric liquid crystals are reduced.

The liquid crystal composition of the present invention is advantageously used for a transmission type ferroelectric liquid crystal display, a reflection type ferroelectric liquid crystal display, a multicolor liquid crystal projection display, a parallel optical computing element, a space light modulation element, a printer head, a light valve, an optical IC card, an optical memory/optical recording element, an E/O element, a steroscopic display device and the like. Especially when the liquid crystal compound is used for various display devices, a light valve, an optical memory/optical recording element and a steroscopic display device, the characteristic property of reduced zigzag defects is effectively utilized. Moreover, when the liquid crystal composition of the present invention is used for various computing elements, an E/O element, a printer head and a light valve, the characteristic property of a short response time is advantageously exerted.

The present invention will now be described in detail with reference to the following examples.

Referential Example 1

Synthesis of β-hydroxynonanoic acid

A solution of 20.0 g of methyl ethyl ketone in 100 ml of dioxane was added dropwise into a solution comprising 210 ml of diethyl carbonate, 12.8 g of sodium hydride dispersed in an oil at a concentration of 60% by weight and 100 ml of dioxane in an argon atmosphere, the mixture was refluxed overnight, and the solvent was removed by distillation. The residue was subjected to distillation under reduced pressure to obtain 20.0 g of ethyl hexylketoacetate. The yield was 62.5% and the boiling point was 83 °C at 0.65 mmHg.

Then, 15 g of the obtained ethyl hexylketoacetate was dissolved in a solution comprising 75 ml of ethanol, 75 ml of distilled water, and 5.02 g of potassium hydroxide, and the solution was stirred at room temperature for 7.5 hours, and thereafter 3 ℓ of distilled water, 360 g of sucrose, and 168 g of dry yeast were added to the solution, and the mixture was shaken at 30 °C for 16 hours and filtered through Celite. The obtained solid was air-dried and extracted with ethyl acetate, and the extract was concentrated. Hydrochloric acid was added to the filtrate so that the pH value was 1, and sodium chloride was added to the filtrate to form a saturated solution, the solution was extracted with chloroform, and the obtained extract and the above-mentioned ethyl acetate extract concentrate were dissolved in diethyl ether. The solution was

extracted two times with a 1N aqueous solution of sodium hydroxide, and hydrochloric acid and sodium chloride were added again to this aqueous solution to form a saturated aqueous solution of sodium chloride having a pH value of 1. The solution was extracted five times with ether, and the ether phase was recovered, washed with a saturated aqueous solution of sodium chloride, and dehydrated on magnesium sulfate. The ether was then evaporated, and the residue was dissolved in n-hexane and recrystallized therefrom to obtain 7.81 g of β-hydroxynonanoic acid [melting point = 49.3 to 50.0 °C, $[\alpha]^{24.5}$ = -20.1° (C = 1.1, $CHCl_3$)].

Referential Example 2

Synthesis of 1-ethyl S- (2)-acetoxybutanedioate of the following formula:

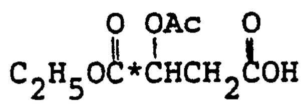

To 50 g of (s)-(-)-malic acid was added 160 ml of acetyl chloride, the mixture was stirred and refluxed at 55 °C for 4 hours, and the solution was concentrated in vacuo. Then, 100 ml of benzene was added to the residue and benzene and acetic acid were removed by distillation in vacuo. The concentrate was cooled to room temperature and 100 ml of absolute ethanol was added thereto, and the mixture was violently stirred while being intermittently cooled. Then the mixture was heated at 70 to 75 °C for 10 minutes and at 50 to 55 °C for 10 hours, the solvent was removed from the mixture by distillation under a reduced pressure, and the residue was separated and purified in a silica gel column by using a methylene chloride/methanol (50/1) mixed solvent as the developing solvent to obtain 50.9 g of 1-ethyl S-(2)-acetoxybutanedioate [melting point = 50 to 51 °C, $[\alpha]^{23}$ = -31.6° *C = 1.42, ethanol)].

Referential Example 3

Synthesis of (2S,5R)-2-hydroxy-5-hexyl-δ-valerolactone of the following formula:

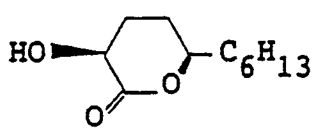

In methanol were dissolved 2.30 g of (R)-β-hydroxynonanoic acid synthesized in Referential Example 1 and 7.90 g of 1-ethyl S-(2)-acetoxybutanedioate synthesized in Referential Example 2, 230 mg of sodium methylate was added to the solution, and the Kolbe electrolysis was carried out under conditions of 20 to 30 °C, 40 V, and 1.5 A for 5 hours by using a constant voltage electrolysis apparatus (Model VE-8 supplied by Yanaco). After the electrolysis, 60 ml of a 3N aqueous solution of sodium hydroxide was added to the hydrolyzed solution, the solution was stirred overnight, methanol was removed, and the residue was washed with ether. The alkaline aqueous solution layer was recovered and hydrochloric acid was added thereto so that the pH value was 1. Then sodium chloride was added to the liquid mixture to form a saturated aqueous solution of sodium chloride, this aqueous solution was extracted with chloroform, the extract was dehydrated on magnesium sulfate, and chloroform was evaporated. The residue left after the evaporation of chloroform was dissolved in 10 ml of benzene, a catalytic amount of p-toluenesulfonic acid was added to the solution, and the mixture was stirred at room temperature for 2 hours and dissolved in ether. The solution was washed three times with a saturated aqueous solution of sodium bicarbonate and once with a saturated aqueous solution of sodium chloride and then dehydrated on magnesium sulfate, and the residue left after removal of ether by evaporation was developed with an n-hexane/ethyl acetate mixed solvent by using a silica gel column to separate and purify the target compound. Recrystallization from an n-hexane/ethyl

acetate mixed solvent gave 310 mg of (2S,5R)-2-hydroxy-5-hexyl-δ-valerolactone. The melting point was 75.5 to 77.0 °C, and the elementary analysis values were as shown below.

Elementary analysis values

Found values: C = 65.84%, H = 10.29%, N = 0.06%

Calculated values: C = 65.97%, H = 10.07%, N = 0%

The specific rotatory power $[\alpha]^{25}$ was +76.8° (C = 1.1 in chloroform). The results of the $^1$H-NMR analysis were as shown below.

$^1$H-NMR, δ ppm:

4.36 (2H), 3.21 (1H), 1.31 (14H), 0.89 (3H)

Referential Example 4

Synthesis of

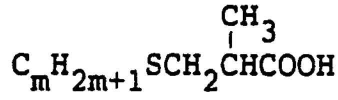

(Compound of m = 4 is illustrated as a typical instance)

In 150 ml of water was dissolved 60 g of sodium hydroxide, and 150 ml of ethanol was added to the solution. Then, 66.5 g (0.5 mole) of methyl L-(+)-β-acetylthio-α-methylpropionate was added to the mixture, the mixture was stirred at room temperature for 1 hour, 109.6 g (0.8 mole) of n-butyl bromide was added, and the mixture was stirred at room temperature overnight. Then, 1N hydrochloric acid was added to the mixture to neutralize the mixture, and the mixture was extracted with diethyl ether. The extract was washed with 1N hydrochloric acid three times and with water three times, and the organic solution was separated and dried on anhydrous magnesium sulfate. An evaporation of diethyl ether gave 56.2 g of optically active 3-butylthio-2-methylpropionic acid, and the obtained optically active 3-butylthio-2-methylpropionic acid was stored on a molecular sieve.

Referential Example 5

Synthesis of 4-bromomethylphenyl-4′-carbonyloxy-5-hexyl-δ-valerolactone of the following formula:

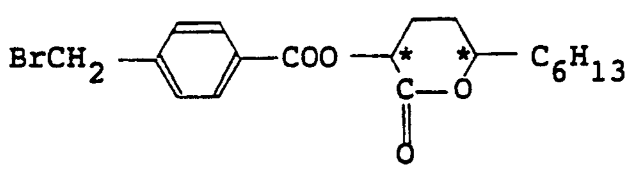

In 500 ml of ether were dissolved 350 mg of (2S,5R)-2-hydroxy-5-hexyl-δ-valerolactone prepared in the same manner as in Referential Example 3, 500 mg of p-bromomethylbenzoic acid and 525 mg of triphenylphosphine, 350 mg of diethyl azodicarboxylate was added dropwise into the solution, and the mixture was stirred overnight to effect reaction. The obtained liquid was concentrated and then separated and purified by the silica gel column chromatography using a benzene/hexane mixed solvent as the developing solvent, and the purified product was recrystallized from an ethyl acetate/ethanol mixed solvent to obtain 100 mg of a crystal of 4-bromomethylphenyl-4′-carbonyloxy-5-hexyl-β-valerolactone.

Referential Example 6

The following composition was prepared as the non-ferroelectric liquid crystal mixture:

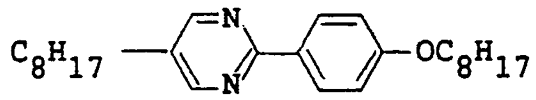

9 mole%

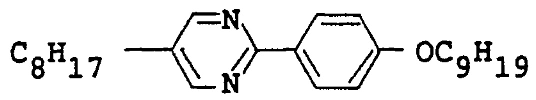

9 mole%

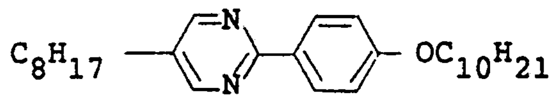

9 mole%

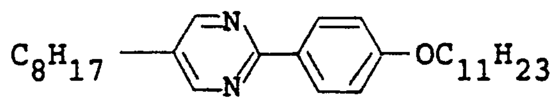

9 mole%

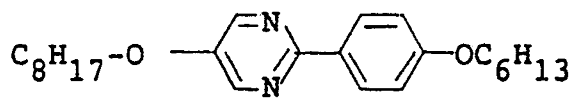

13.3 mole%

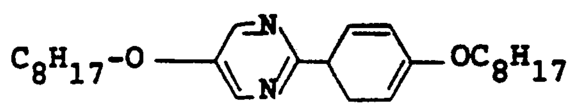

13.3 mole%

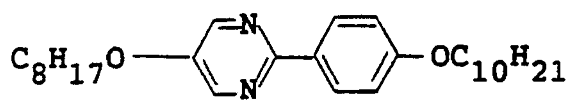

13.3 mole%

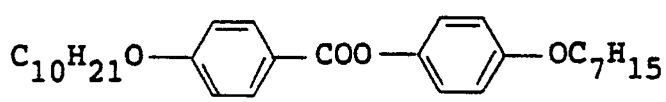

8 mole%

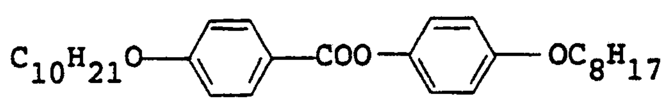

8 mole%

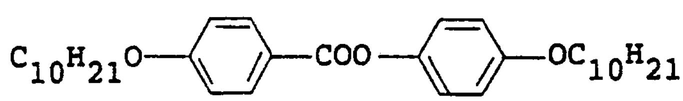

8 mole%

Example 1

Synthesis of (2R,5R,2‴S)-2-[{4″-(3‴-hexylthio-2‴-methylpropanoyloxy)biphenyl}-4′-carbonyloxyl]-5-hexyl-δ-valerolactone of the following formula:

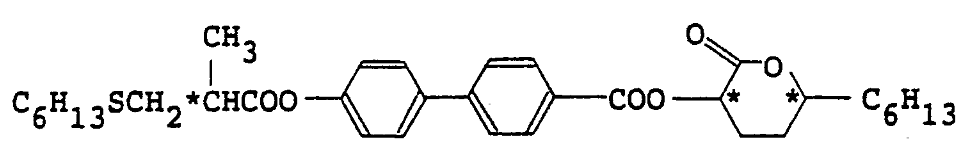

To 2 g of 4-hydroxy-4′-biphenylcarboxylic acid were added 1.6 g of benzyl bromide and 4.2 g of

potassium carbonate, and the mixture was refluxed for 20 hours by using acetone as the solvent. Acetone was removed by distillation, the residue extracted with chloroform, and the extract washed with water two times, with 1N hydrochloric acid two times, and again with water two times. The organic phase was separated and dried on anhydrous magnesium sulfate, and the organic solvent was evaporated and the residue recrystallized from ethanol to obtain 2.1 g of a white crystal. The crystal was dissolved in hot ethanol, 40 ml of a 2N solution of potassium hydroxide was added to the solution, and the mixture was stirred at 60°C for 5 hours. The solution was made acidic by an addition of hydrochloric acid, and the precipitated crystal was recovered by filtration, washed with water and dried to obtain 1.9 g of 4′-benzyloxybiphenylcarboxylic acid.

In 20 ml of benzene were dissolved 350 mg of (2S,5R)-2-hydroxy-5-hexyl-δ-valerolactone prepared in the same manner as in Referential Example 3, 532 mg of 4′-benzyloxy-biphenylcarboxylic acid and 460 mg of triphenylphosphine, and 200 mg of diethyl azodicarboxylate was added dropwise into the solution and the mixture stirred overnight to effect a reaction. The thus-obtained solution was concentrated and then separated and purified by the silica gel column chromatography using a benzene/hexane mixed solvent as the developing solvent, and the purified product was recrystallized from an ethyl acetate/ethanol mixed solvent to obtain 420 mg of a white crystal.

The crystal was dissolved in a 2:1 ethyl acetate-ethanol mixed solvent, 250 mg of 5% palladium-carbon was added to the solution, and a catalytic reduction was carried out under a hydrogen pressure of 1.5 $kg/cm^2$ to remove the benzyl group. Then, 200 mg of the obtained crystal, 120 mg of L-(+)-3-hexylthio-2-methylpropionic acid synthesized in the same manner as in Referential Example 4 and 125 mg of dicyclohexylcarbodiimide were dissolved in 15 ml of methylene chloride, the solution was stirred overnight, floating substances were removed by filtration, and 15 ml of methylene chloride was added to the solution. Then, the solution was washed with a saturated solution of sodium hydrogencarbonate two times and with water two times, and the organic phase was separated and dried on anhydrous magnesium sulfate. The organic solvent was evaporated and a product was isolated by a silica gel column using a benzene/n-hexane mixed solvent to obtain a white powder. The product was recrystallized from an n-hexane/ethanol mixed solvent to obtain 170 mg of (2R,5R,2‴S)-2-[{4″-(3‴-hexylthio-2‴-methylpropanoyloxy)biphenyl}-4′-carbonyloxy]-5-hexyl-δ-valerolactone.

From the NMR spectrum and infrared absorption spectrum of the obtained compound, it was confirmed that the obtained compound was the target compound. The NMR spectrum of the compound is shown in Fig. 1. The melting point of the compound was 86°C.

## Examples 2, 3 and 4

Following the same procedure as in Example 1, compounds shown in Table 1 were synthesized. The melting points of these compounds are shown in Table 1.

## Example 5

Synthesis of (2R,5R,2″S)-2-[4′-(3″-hexylthio-2″-methylpropanoyloxy)benzoyloxyl]-5-hexyl-δ-valerolactone of the following formula:

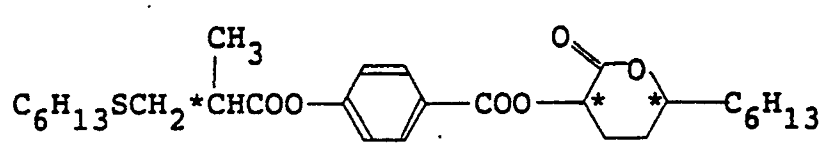

In 10 ml of methylene chloride were dissolved 70 mg of (2R,5R)-2-(4′-hydroxybenzoyloxy)-5-hexyl-δ-valerolactone synthesized in the same manner as in Example 1 except that 4-hydroxybenzoic acid was used instead of 4′-hydroxybiphenylcarboxylic acid, 50 mg of L-(+)-3-hexylthio-2-methylpropionic acid, and 50 mg of dicyclohexylcarbodiimide, and the solution was stirred overnight. Floating substances were removed by filtration, 10 ml of methylene chloride was added to the reacted solution and the solution was washed with a saturated solution of sodium hydrogencarbonate two times and with water two times. The organic phase was separated and dried on anhydrous magnesium sulfate. The organic solvent was

evaporated, and a product was isolated by a silica gel column using benzene/n-hexane as the developing solvent and was recrystallized from an n-hexane/ethanol mixed solvent to obtain 50 mg of (2R,5R,2″S)-2-[4,-(3″-hexylthio-2″-methylpropanoyloxy)benzoyloxy]-5-hexyl-δ-valerolactone.

From the NMR spectrum and infrared absorption spectrum of the obtained compound, it was confirmed that the obtained compound was the target compound. This compound has a melting point of 51 °C.

Examples 6 and 7

Following the same procedure as in Example 5, compounds shown in Table 1 were synthesized. The melting points of these compounds are shown in Table 1.

Example 8

Synthesis of a compound of the following formula:

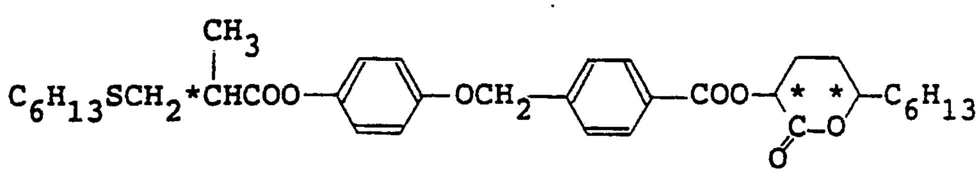

To 100 ml of dimethylformamide were added 240 mg of sodium hydride and then 296 mg of L-(+)-3-hexylthio-2-methylpropanoyloxycarbonylphenol, and the mixture was stirred at room temperature for one hour. Then a solution of 397 mg of 4-bromomethylphenyl-4′-carbonyloxy-5-hexyl-δ-valerolactone synthesized in a manner similar to in Referential Example 5, in 20 ml of dimethylformamide, was added dropwise to the reacted mixture and the reacted mixture was stirred for two hours. The reaction product was extracted with ether and the obtained solution was separated and purified by the silica gel column chromatography using benzene-hexane as the developing solvent, and the purified product was recrystallized from an ethyl acetate-ethanol mixed solvent to obtain a crystal.

From the NMR spectrum and infrared absorption spectrum of the obtained crystal, it was confirmed that the obtained crystal was the target compound. The melting point of the compound was 71 °C.

Examples 9, 10 and 11

Following the same procedure as in Example 8, compounds shown in Table 1 were synthesized. The melting points of these compounds are shown in Table 1.

Table 1

| Example No. | Compound | Melting point (°C) |
|---|---|---|
| 2 | 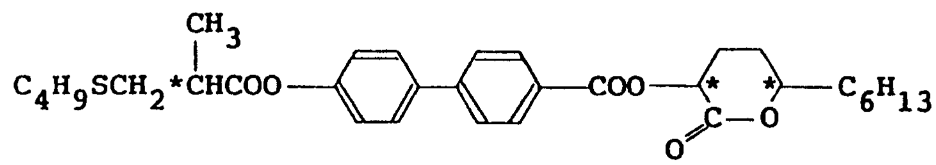 | 83 |
| 3 | 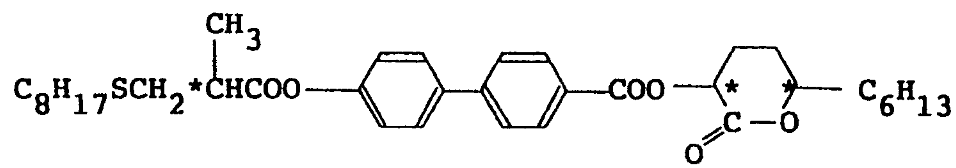 | 89 |
| 4 | 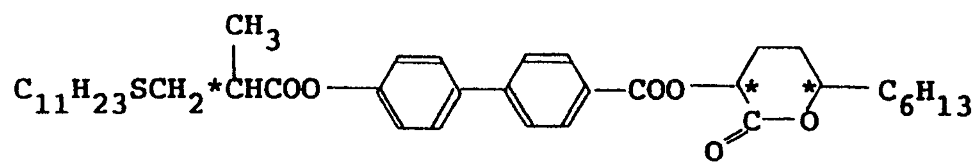 | 91 |
| 6 | 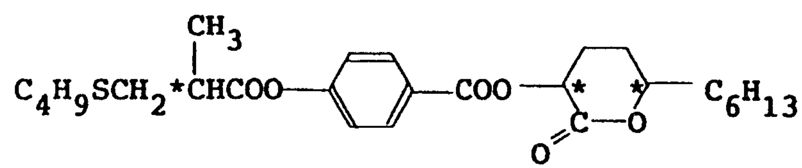 | 48 |

Table 1 (Continued)

| Example No. | Compound | Melting point (°C) |
|---|---|---|
| 7 | 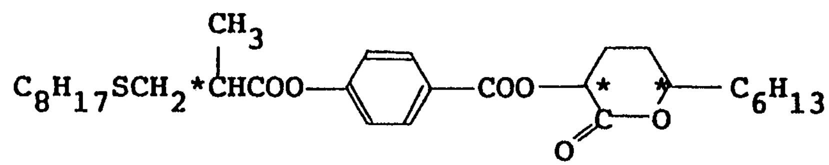 | 53 |
| 9 | 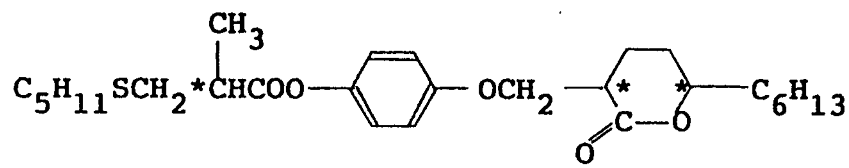 | 71 |
| 10 | 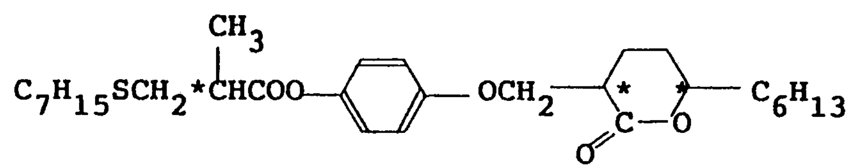 | 73 |
| 11 | 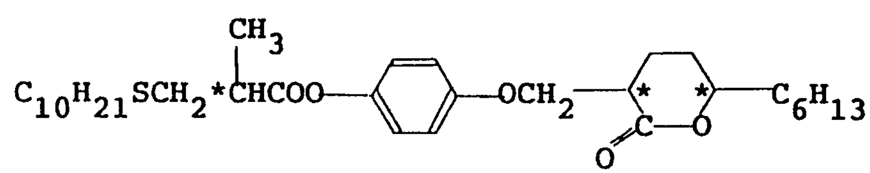 | 76 |

Example 12

A ferroelectric liquid crystal composition was prepared by mixing 2 molar parts of the compound synthesized in Example 1 with 98 molar parts of the liquid crystal composition prepared in Referential Example 6. The phase transition temperatures of the obtained composition were as follows:

$$\text{Cryst} \overset{3°C}{\longleftrightarrow} \text{Sm*C} \overset{68°C}{\longleftrightarrow} \text{SmA} \overset{75°C}{\longleftrightarrow} \text{Ch} \overset{80°C}{\longleftrightarrow} \text{Iso}$$

The helical pitch of the Ch phase at 76° C was 12 µm.

The composition was sealed in a liquid crystal cell having a cell gap of 2 µm, which was provided with a transparent electroconductive glass (ITO glass), which had been parallel-oriented by coating a polyimide (PIX-5400 supplied by Hitachi Chemical) as the orienting agent on the surface and subjecting the surface to a rubbing treatment. The constructed liquid crystal element was cooled at a rate of 1° C/min from 90° C whereby the phase was converted to the Sm*C phase through the SmA phase. A good molecular orientation of the liquid crystal was obtained.

Then, a square wave (10 Hz) of ±10 V was applied to the cell at 25° C. The cell showed an electrooptical response, and the response time required for the light transmission to change to 90% from 0% was 300 µsec. The tilt angle at 25° C was 24°.

Example 13

A ferroelectric liquid crystal composition was prepared by mixing 5 molar parts of the compound synthesized in Example 1 with 95 molar parts of the liquid crystal mixture prepared in Referential Example 6. The phase transition temperatures of the obtained composition were as shown below.

$$\text{Cryst} \overset{2°C}{\longleftrightarrow} \text{Sm*C} \overset{68°C}{\longleftrightarrow} \text{SmA} \overset{72°C}{\longleftrightarrow} \text{Ch} \overset{79°C}{\longleftrightarrow} \text{Iso}$$

The composition was sealed in a liquid crystal cell having a cell gap of 2 µm, which was provided with a transparent electroconductive glass (ITO glass), which had been parallel-oriented by coating a polyimide (PIX-5400 supplied by Hitachi Chemical) as the orienting agent on the surface and subjecting the surface to a rubbing treatment. The constructed liquid crystal element was cooled at a rate of 1° C/min from 90° C and the phase was converted to the Sm*C phase through the SmA phase. A good molecular orientation of the liquid crystal was obtained.

Then, a square wave (10 Hz) of ±10 V was applied to the cell at 25° C. The cell showed an electrooptical response, and the response time required for the light transmission to change to 90% from 0% was 160 µsec. The tilt angle at 25° C was 26°.

Examples 14 through 25

Using each of the compounds described in Examples 2 through 11, a ferroelectric liquid crystal composition was prepared in the same manner as described in Example 12 or 13. The phase transition temperature, response speed and tilt angle at 25° C thereof are shown in Table 2.

Table 2

| Example No. | Example in which compound is prepared | Amount (% by mole) | Phase transition | Response speed (μsec) | Tilt angle (degree) |
|---|---|---|---|---|---|
| 14 | 2 | 2 | Cryst $\xleftrightarrow{2^\circ C}$ Sm*C $\xleftrightarrow{69^\circ C}$ SmA $\xleftrightarrow{76^\circ C}$ Ch $\xleftrightarrow{81^\circ C}$ Iso | 290 | 24 |
| 15 | 3 | 2 | Cryst $\xleftrightarrow{3^\circ C}$ Sm*C $\xleftrightarrow{68^\circ C}$ SmA $\xleftrightarrow{75^\circ C}$ Ch $\xleftrightarrow{80^\circ C}$ Iso | 310 | 24 |
| 16 | 4 | 2 | Cryst $\xleftrightarrow{3^\circ C}$ Sm*C $\xleftrightarrow{67^\circ C}$ SmA $\xleftrightarrow{75^\circ C}$ Ch $\xleftrightarrow{79^\circ C}$ Iso | 320 | 24 |
| 17 | 5 | 2 | Cryst $\xleftrightarrow{1^\circ C}$ Sm*C $\xleftrightarrow{67^\circ C}$ SmA $\xleftrightarrow{74^\circ C}$ Ch $\xleftrightarrow{79^\circ C}$ Iso | 230 | 24 |
| 18 | 6 | 2 | Cryst $\xleftrightarrow{1^\circ C}$ Sm*C $\xleftrightarrow{67^\circ C}$ SmA $\xleftrightarrow{74^\circ C}$ Ch $\xleftrightarrow{79^\circ C}$ Iso | 230 | 19 |
| 19 | 7 | 2 | Cryst $\xleftrightarrow{2^\circ C}$ Sm*C $\xleftrightarrow{66^\circ C}$ SmA $\xleftrightarrow{73^\circ C}$ Ch $\xleftrightarrow{78^\circ C}$ Iso | 250 | 19 |

Table 2 (Continued)

| Example No. | Example in which compound is prepared | Amount (% by mole) | Phase transition | Response speed (μsec) | Tilt angle (degree) |
|---|---|---|---|---|---|
| 20 | 8 | 2 | Cryst $\xleftrightarrow{2°C}$ Sm*C $\xleftrightarrow{69°C}$ SmA $\xleftrightarrow{75°C}$ Ch $\xleftrightarrow{80°C}$ Iso | 190 | 22 |
| 21 | 9 | 2 | Cryst $\xleftrightarrow{3°C}$ Sm*C $\xleftrightarrow{68°C}$ SmA $\xleftrightarrow{75°C}$ Ch $\xleftrightarrow{80°C}$ Iso | 180 | 22 |
| 22 | 10 | 2 | Cryst $\xleftrightarrow{3°C}$ Sm*C $\xleftrightarrow{69°C}$ SmA $\xleftrightarrow{76°C}$ Ch $\xleftrightarrow{79°C}$ Iso | 190 | 22 |
| 23 | 11 | 2 | Cryst $\xleftrightarrow{3°C}$ Sm*C $\xleftrightarrow{69°C}$ SmA $\xleftrightarrow{76°C}$ Ch $\xleftrightarrow{79°C}$ Iso | 200 | 22 |
| 24 | 10 | 5 | Cryst $\xleftrightarrow{4°C}$ Sm*C $\xleftrightarrow{67°C}$ SmA $\xleftrightarrow{76°C}$ Ch $\xleftrightarrow{80°C}$ Iso | 130 | 23 |
| 25 | 11 | 5 | Cryst $\xleftrightarrow{4°C}$ Sm*C $\xleftrightarrow{67°C}$ SmA $\xleftrightarrow{76°C}$ Ch $\xleftrightarrow{80°C}$ Iso | 140 | 23 |

The optically active substance of the present invention is chemically stable and is not colored, and has an excellent light stability. Furthermore, a liquid crystal composition comprising this optically active substance shows ferroelectric characteristics over a broad temperature range, and is further characterized in that the response speed is fast. Furthermore, since an optically active branched alkyl chain is introduced on the side opposite to the valerolactone group, the helical pitch of the Sm*C phase becomes long and the melting point becomes low.

Moreover, the optically active substance of the present invention is characterized in that the zigzag defects often observed in conventional ferroelectric liquid crystals are reduced.

## Claims

1. An optically active substance having a δ-valerolactone ring, which is represented by the following general formula (1):

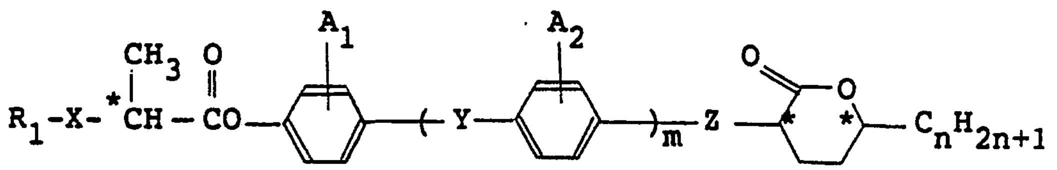

(1)

wherein X represents -O-, $-OCH_2-$ or $-SCH_2-$, $R_1$ represents an alkyl group having 1 to 18 carbon atoms, n is an integer of from 1 to 14, m is 1 or 0, Y represents a direct bond, -OCO-, $-CO_2-$, $-CH_2O-$ or $-OCH_2-$, Z represents -O-, $-CO_2-$ or $-CH_2O$, $A_1$ and $A_2$ independently represent hydrogen, fluorine, chlorine or a cyano group, and the carbon atom marked with "*" indicates an asymmetric carbon atom.

2. An optically active substance according to claim 1 and having the formula shown in any of the examples.

3. A liquid crystal composition comprising an optically active substance according to claim 1 or claim 2.

Fig.1